# EUROPÄISCHE PATENTSCHRIFT

(11) **EP 0 399 633 B1**
(45) Veröffentlichungstag und Bekanntmachung des Hinweises auf die Patenterteilung: **30.11.1994**
(21) Anmeldenummer: 90250136.0
(22) Anmeldetag: 25.05.1990
(51) Int. Cl.: C07C 69/18, C07C 69/16, C07C 59/46, C07C 33/14, C07C 69/732, C07D 309/30, A61K 31/557, C07C 405/00

(54) **Neue Leukotrien B4-Derivate Verfahren zu ihrer Herstellung und ihre Verwendung als Arzneimittel**
Leukotriene B4 derivatives, process for their preparation and their use as medicines
Dérivés de leukotriène B4, procédé pour leur préparation et leur utilisation comme médicaments

(30) Priorität: 26.05.1989 DE 3917597
(43) Veröffentlichungstag der Anmeldung: 28.11.1990
(73) Patentinhaber: SCHERING AKTIENGESELLSCHAFT, 13353 Berlin (DE)
(72) Erfinder: Skuballa, Werner, Dr., D-1000 Berlin 28 (DE); Buchmann, Bernd, Dr., D-1000 Berlin 21 (DE); Heindl, Joseph, Dr., D-1000 Berlin 38 (DE); Fröhlich, Wolfgang, Dr., D-1000 Berlin 31 (DE); Ekerdt, Roland, Dr., D-1000 Berlin 28 (DE)

(56) Entgegenhaltungen:
- TETRAHEDRON LETTERS, Band 29, Nr. 2, 1988, Seiten 143-146, Pergamon Journals Ltd, Oxford, GB; J. MORRIS et al.: "Synthesis of novel antagonists of leukotriene B4"
- PROSTAGLANDINS, Band 35, Nr. 5, Mai 1988, Seiten 723-731, Stoneham, MA, US; P. BORGEAT et al.: "Rearrangement of 5S,12S-dihydroxy-6,8,10,14- (E,Z,E,Z)-eicosatetraenoic acid during gas chromatography: Formation of a cyclohexadiene derivative"

## Beschreibung

Die Erfindung betrifft neue Leukotrien-B₄-Derivate, Verfahren zu ihrer Herstellung sowie ihre Verwendung als Arzneimittel.
Leukotrien B₄ (LTB₄) wurde 1979 von B. Samuelsson und Mitarbeiter als Metabolit der Arachidonsäure entdeckt. Bei der Biosynthese wird durch das Enzym 5-Lipoxygenase zunächst als zentrales Zwischenprodukt das Leukotrien A₄ gebildet, das dann durch eine spezifische Hydrolase in das LTB₄ umgewandelt wird.

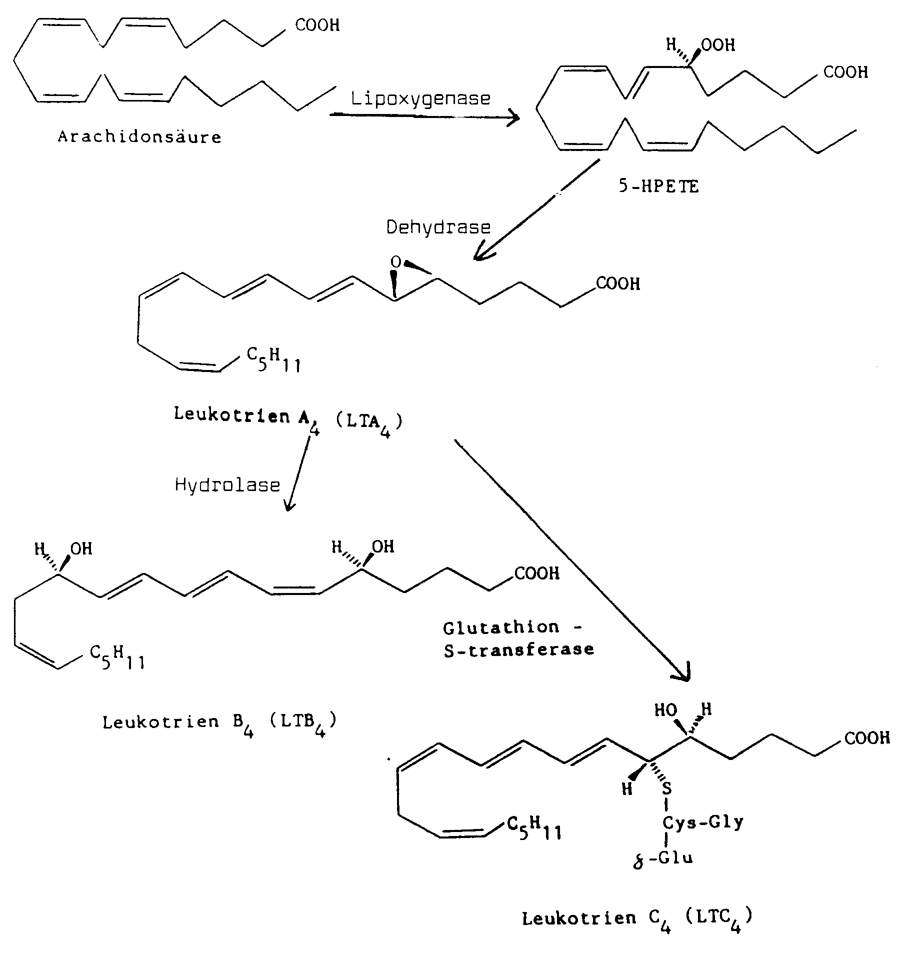

Die Nomenklatur der Leukotriene kann folgenden Arbeiten entnommen werden: a) B. Samuelsson et al. , Prostaglandins 19, 645 (1980); 17, 785 (1979). b) C.N. Serhan et al., Prostaglandins 34, 201 (1987).

Die physiologische und insbesondere die pathophysiologische Bedeutung des Leukotrien B₄ wird in einigen neueren Arbeiten zusammengefaßt: a) The Leukotrienes, Chemistry and Biology eds. L.W. Chakrin, D.M. Bailey, Academic Press 1984. b) J.W. Gillard et al. , Drugs of the Future 12, 453 (1987). c) B. Samuelsson et al. , Science 237, 1171 (1987). d) C.W. Parker, Drug Development Research 10, 277 (19871. Hierzu ergibt sich, daß LTB₄ ein wichtiger Entzündungsmediator für entzündliche Erkrankungen ist, bei denen Leukocyten in das erkrankte Gewebe einwandern.
Von LTB₄ weiß man, daß es die Adhäsion von Leukozyten an die Blutgefäßwand verursacht. LTB₄ ist chemotaktisch wirksam, d.h.es löst eine gerichtete Wanderung von Leukozyten in Richtung eines Gradienten steigender Konzentration aus. Außerdem verändert es aufgrund seiner chemotaktischen Aktivität indirekt die vasculäre Permeabilität, wobei ein Synergismus mit Prostaglandin E₂ beobachtet wird. LTB₄ spielt offensichtlich bei entzündlichen, allergischen und immunologischen Prozessen eine entscheidende Rolle.

Leukotriene und insbesondere LTB₄ sind an Hauterkrankungen beteiligt, die mit entzündlichen Prozessen (erhöhte Gefäßpermeabilität und Ödembildung, Zellinfiltration), erhöhter Proliferation der Hautzellen und Juckreiz einhergehen, wie beispielsweise bei Ekzemen, Erythemen, Psoriasis, Pruritus und Akne. Pathologisch erhöhte Leukotrien-Konzentrationen sind an der Entstehung vieler Dermatitiden entweder ursächlich beteiligt, oder es besteht ein Zusammenhang zwischen der Persistenz der Dermatitiden und den Leukotrienen. Deutlich erhöhte Leukotrienkonzentrationen wurden beispielsweise in der Haut von Patienten mit Psoriasis oder atopischer Dermatitis gemessen.
Weiterhin sind Leukotriene und LTB₄ insbesondere bei Arthritis, chronischer Lungenerkrankungen (z. B. Asthma), Rhi nitis und entzündlichen Darmerkrankungen beteiligt.
Antagonisten gegen LTB₄-Rezeptoren oder -Inhibitoren jener Enzyme, die an der Synthese des LTB₄ beteiligt sind, sollten als spezifische Heilmittel, besonders gegen Krankheiten, die mit Entzündungen und allergischen Reaktionen einhergehen, wirksam sein.

So werden von Joel Morris und Mitarbeitern (Tetrahedron Letters 29, 1988, 143-146) Verbindungen beschrieben, die sich vom LTB₄ dadurch unterscheiden, daß die Kohlenstoffatome 7-9 des LTB₄ durch einen 2,6-disubstituierten Pyridinring ersetzt sind. Diese Substanzen sind stabile LTB₄-Aritagonisten und sie besitzen eine antiinflammatorische Wirksamkeit.

Neben therapeutischen Möglichkeiten, die sich aus einer Antagonisierung des LTB₄ mit LTB₄-Analoga ableiten lassen, konnte kürzlich auch die Nützlichkeit und potentielle Anwendung von Leukotrien B₄-Agonisten zur Behandlung von Pilzerkrankungen der Haut gezeigt werden (H. Kayama, Prostaglandins 34, 797 (1988)).

Es wurde nun gefunden, daß der Einbau der chemisch und metabolisch labilen cis-Δ^{6,7}-Doppelbindung des LTB₄ in einen cis-1,2-substituierten Cyclohexylring oder einen trans-1,2-substituierten Cyclohexylring zu einer Stabilisierung führt, wobei insbesondere durch weitere Derivatisierung der funktionellen Gruppen, LTB₄-Derivate erhalten werden, die die Wirkung des natürlichen LTB₄ stark antagonisieren. Das heißt, die Erfindung umfaßt LTB₄ Analoga, die als Antagonisten wirken können.

Die Erfindung betrifft Verbindungen der Formel I, 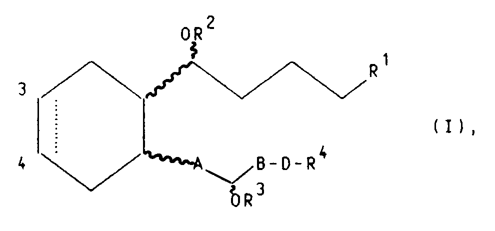
wobei die Reste folgende Bedeutungen haben: 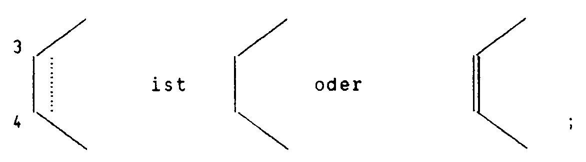
- R¹: ist CH₂OH, CH₃, CF₃, COOR⁵ mit R⁵ in der Bedeutung eines Wasserstoffatoms, eines Alkylrestes mit 1-10 C-Atomen, eines Cycloalkylrestes mit 3-10 C-Atomen, eines gegebenenfalls durch 1-3 Chlor, Brom, Phenyl, C₁₋₄-Alkyl, C₁₋₄-Alkoxy, Chlormethyl, Fluormethyl, Trifluormethyl, Carboxy oder Hydroxy substituierten Arylrestes mit 6-10 C-Atomen, eines -CH₂-CO-Aryl-Restes mit 6-10 C-Atomen für Aryl oder eines 5-6-gliedrigen aromatischen heterocyclischen Restes mit wenigstens 1 Heteroatom oder
- R¹: ist CONHR⁶ mit R⁶ in der Bedeutung eines Alkanoyl- oder Alkansulfonylrestes mit 1-10 C-Atomen oder des Restes R⁵;
- A: ist eine trans, trans-CH=CH-CH=CH- oder Tetramethylengruppe;
- B: ist eine geradkettige oder verzweigtkettige gesättigte oder ungesättigte Alkylengruppe mit bis zu 10 C-Atomen, die gegebenenfalls durch Fluor substituiert sein kann, oder die Gruppe 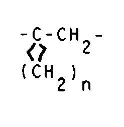 mit n=1-3;
- D: ist eine Direktbindung, Sauerstoff, Schwefel, eine -C≡C-Gruppe oder eine -CH=CR⁷-Gruppe mit R⁷ als Wasserstoff, C₁₋₅-Alkyl, Chlor, Brom oder 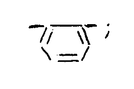
- B: und D sind gemeinsam eine Direktbindung;
- R² und R³: sind gleich oder verschieden und bedeuten Wasserstoff oder einen organischen Säurerest mit 1-15 C-Atomen;
- R¹ und R²: sind gemeinsam eine Carbonylgruppe;
- R⁴: ist ein Wasserstoffatom, C₁₋₁₀-Alkyl, das gegebenenfalls durch Chlor oder Brom substituiert sein kann, Cycloalkyl mit 3-10 C-Atomen , ein gegebenenfalls durch 1-3 Chlor, Brom, Phenyl, C₁₋₄-Alkyl, C₁₋₄-Alkoxy, Chlormethyl, Fluormethyl, Trifluormethyl, Carboxy oder Hydroxy substituierter Arylrest mit 6-10 C-Atomen oder ein 5-6-gliedriger aromatischer heterocyclischer Rest mit wenigstens 1-Heteroatom und falls
- R⁵: ein Wasserstoffatom bedeutet, deren Salze mit physiologisch vertraglichen Basen und deren Cyclodextrinclathrate.

Die Gruppen OR² und OR³ können α- oder β-ständig sein. Die Formel I umfaßt sowohl Racemate als auch die möglichen reinen Diastereomeren und Enantiomeren.

Als Alkylgruppen R⁵ kommen gerad- oder verzweigtkettige Alkylgruppen mit 1-10 C-Atomen in Betracht, wie beispielsweise Methyl, Ethyl, Propyl, Butyl, lsobutyl, tert.-Butyl, Pentyl, Neopentyl, Hexyl, Heptyl, Decyl. Die Alkylgruppen R⁵ können gegebenenfalls ein- bis mehrfach substituiert sein durch Halogenatome, Alkoxygruppen, gegebenenfalls substituierte Aryl- bzw. Aroylgruppen mit 6-10 C-Atomen (Substitution s. unter Aryl R⁵), Dialkylamino und Trialkylammonium mit 1-4 C-Atomen im Alkyl-Teil, wobei die einfache Substitution bevorzugt sein soll. Als Substituenten seien beispielsweise genannt Fluor, Chlor oder Brom, Phenyl, Dimethylamino, Diethylamino, Methoxy, Ethoxy. Als bevorzugte Alkylgruppen R⁵ sind solche mit 1-4 C-Atomen zu nennen.

Als Arylgruppen R⁵ kommen sowohl substituierte wie auch unsubstituierte Arylgruppen mit 6-10 C-Atomen in Betracht, wie beispielsweise Phenyl, 1-Naphthyl und 2-Naphthyl, die jeweils substituiert sein können durch 1-3 Halogenatome (F, Cl, Br), eine Phenylgruppe, 1-3 Alkylgruppen mit jeweils 1-4 C-Atomen, eine Chlormethyl-, Fluormethyl-, Trifluormethyl, - Carboxyl-, Hydroxy- oder Alkoxygruppe mit 1-4 C-Atomen. Bevorzugte Substituenten in 3- und 4-Stellung am Phen ylring sind zum Beispiel Fluor, Chlor, Alkoxy oder Trifluormethyl, in 4-Stellung dagegen Hydroxy.

Die Cycloalkylgruppe R⁵ kann im Ring 3-10, vorzugsweise 5 und 6 Kohlenstoffatome enthalten. Die Ringe können durch Alkylgruppen mit 1-4 Kohlenstoffatomen substituiert sein. Beispielsweise seien genannt Cyclopentyl-, Cyclohexyl, Methylcyclohexyl.

Als heterocyclische Gruppen R⁵ kommen 5- und 6-gliedrige aromatische Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl, 2-Tetrazolyl u.a.

Als Säurerest R⁶ kommen physiologisch verträgliche Säurereste in Frage. Bevorzugte Säuren sind organische Carbonsäuren und Sulfonsäuren mit 1-15 Kohlenstoff atomen, die der aliphatischen, cycloaliphatischen, aromatischen, aromatischaliphatischen und heterocyclischen Reihe angehören. Diese Säuren können gesättigte, ungesättigte und/oder mehrbasisch und/oder in üblicher Weise substituiert sein. Als Beispiele für die Substituenten seien C₁₋₄-Alkyl-, Hydroxy-, C₁₋₄-Alkoxy, Oxo- oder Aminogruppen oder Halogenatome (F, Cl, Br) erwähnt. Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyesigsäure, Methoxyessigsäure, Ethoxyessigsäure, Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen (F, Cl, Br) oder Trifluormethyl-, Hydroxy, C₁₋₄-Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Acylreste und Alkansulfonylreste werden solche mit bis zu 10 Kohlenstoffatomen betrachtet. Als Sulfonsäuren kommen beispielsweise Methansulfonsäure, Ethansulfonsäure, Isopropansulfonsäure, β-Chlorethansulfonsäure, Butansulfonsäure, Cyclopentansulfonsäure, Cyclohexansulfonsäure, Benzolsulfonsäure, p-Toluolsulfonsäure, p-Chlorbenzolsulfonsäure, N,N-Dimethylaminosulfonsäure, N,N-Diethylaminosulfonsäure, N,N-Bis-(β-chlorethyl)-aminosulfonsäure, N,N-Diisobutylaminosulfonsäure, N,N-Dibutylaminosulfonsäure, Pyrrolidino-, Piperidino-, Piperazino-, N-Methylpiperazino- und Morpholinosulfonsäure in Frage.

Als Alkylgruppen R⁴ kommen gerad- und verzweigtkettige, gesättigte und ungesattigte Alkylreste, vorzugsweise gesättigte, mit 1-14, insbesondere 1-10 C-Atomen, in Frage, die gegebenenfalls durch gegebenenfalls substituiertes Phenyl (Substitution s. unter Aryl R⁵) substituiert sein können. Beispielsweise genannt seien Methyl-, Ethyl-, Propyl-, Butyl-, Isobutyl-, tert.,-Butyl-, Pentyl-Hexyl-, Heptyl-, Octyl-, Butenyl-, Isobutenyl-, Propenyl-, Pentenyl-, Benzyl-, m- und p-Chlorbenzylgruppen. Sind die Alkylgruppen R₄ Halogen-substituiert, kommen als Halogene Fluor, Chlor und Brom in Frage.

Als Beispiel für Halogen-substituierte Alkylgruppen R⁴ kommen Alkyle mit terminalen Trifluormethylgruppen in Betracht.

Die Cycloalkylgruppe R⁴ kann im Ring 3-10, vorzugsweise 3-6 Kohlenstoffatome enthalten. Die Ringe können durch Alkylgruppen mit 1-4 Kohlenstoffatomen substituiert sein. Beispielsweise genannt seinen Cyclopropyl, Cyclobutyl, Cyclopentyl, Cyclohexyl, Methyl-cyclohexyl.

Als substituierte bzw. unsubstituierte Arylgruppen R⁴ kommen beispielsweise in Betracht: Phenyl, 1-Naphthyl und 2-Naphthyl, die jeweils substituiert sein können durch 1-3 Halogenatome, eine Phenylgruppe, 1-3 Alkylgruppen mit jeweils 1-4 C-Atomen eine Chlormethyl-, Fluormethyl-, Trifluormethyl-, Carboxyl-, C₁-C₄-Alkoxy- oder Hydroxygruppe. Bevorzugt ist die Substitution in 3- und 4-Stellung am Phenylring zum Beispiel durch Fluor, Chlor, Alkoxy oder Trifluormethyl oder in 4-Stellung durch Hydroxy.

Als heterocyclische aromatische Gruppen R⁴ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl, u.a.

Als Alkylengruppe B kommen geradkettige oder verzweigtkettige, gesättigte oder ungesättigte Alkylenreste, vorzugsweise gesättigte mit 1-10, insbesondere mit 1-5 C-Atomen, in Frage, die gegebenenfalls durch Fluoratome substituiert sein können. Beispielsweise seien genannt: Methylen, Fluormethylen, Difluormethylen, Ethylen, 1,2-Propylen, Ethylethylen, Trimethylen, Tetramethylen, pentamethylen, 1,2-Difluorethylen, 1-Fluorethylen, 1-Methyltetramethylen, 1-Methyl-tri-methylen, 1-Methylen-ethylen, 1-Methylen-tetramethylen.
Die Alkylengrrnppe B kann weiterhin die Gruppe 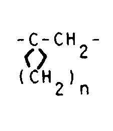
darstellen, wobei n=1-3, bevorzugt 2-3 bedeutet.

Als Säurereste R² und R³ kommen physiologisch verträgliche Säurereste in Frage. Bevorzugte Säuren sind organische Carbonsäuren und Sulfonsäuren mit 1-15 Kohlenstoffatomen, die der aliphatischen, cyclo-aliphatischen, aromatischen, aromatisch-aliphatischen oder heterocyclischen Reihe angehören. Diese Säuren können gesättigt, ungesättigt und/oder mehrbasisch und/oder in üblicher Weise substituiert sein. Als Beispiele für die Substituenten seien C₁₋₄-Alkyl-, Hydroxy-, C₁₋₄-Alkoxy, Oxo- oder Aminogruppen oder Halogenatome (F, Cl, Br) erwähnt.

Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Hyristinsäure, Pentadecylsäure, Trimethylessigsäure, Diethylessigsäure, tert.-Butylessigsäure, Cyclopentylessigsäure, Cyclohexylessigsäure, Cyclohexancarbonsäure, Phenylessigsäure, Phenoxyesigsäure, Methoxyessigsäure, Ethoxyessigsäure, Mono-, Di- und Trichloressigsäure, Aminoessigsäure, DiethylaminoessigSäure, Piperidinoessigsäure, Morpholinoessigsäure, Milchsäure, Bernsteinsäure, Adipinsäure, Benzoesäure, mit Halogen-(F, Cl, Br), Trifluormethyl-, Hydroxy-, C₁₋₄-Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Säurereste R² und R³ werden Acylreste mit bis zu 10 Kohlenstoffatomen betrachtet.
R⁷ als C₁₋₅-Alkyl bedeutet geradkettige oder verzweigtkettige Alkylreste wie sie für R⁴ bzw. R⁵ bereits genannt wurden. Bevorzugte Alkylreste R⁷ sind Methyl, Ethyl, Propyl und Isopropyl.

Zur Salzbildung sind anorganische und organische Basen geeignet, wie sie dem Fachmann zur Bildung physiologisch verträglicher Salze bekannt sind. Beispielsweise seien genannt Alkalihydroxide, wie Natrium- und Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin usw.

Bevorzugte Verbindungen der vorliegenden Erfindung sind Verbindungen der Formel I, wobei die Reste die folgende Bedeutung haben: 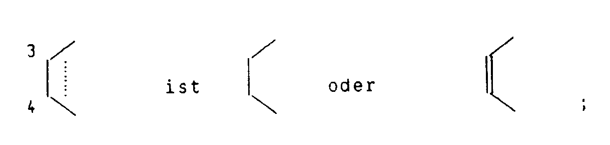
- R¹: ist CH₂OH, COOR⁵ mit R⁵ in der Bedeutung eines Wasserstoffatoms, eines Alkylrestes mit 1-10 C-Atomen, eines Cycloalkylrestes mit 5-6 C-Atomen, eines gegebenenfalls durch 1-2 Chlor, Brom, Phenyl, C₁₋₄-Alkyl, C₁₋₄-Alkoxy, Chlormethyl, Fluormethyl, Trifluormethyl, Carboxy oder Hydroxy substituierten Phenylrestes oder
- R¹: ist CONHR⁶ mit R⁶ in der Bedeutung eines Alkanoyl- oder Alkansulfonylrestes mit 1-10 C-Atomen oder des Restes R⁵;
- A: ist eine trans, trans-CH=CH-CH=CH- oder Tetramethylengruppe;
- B: ist eine geradkettige oder verzweigtkettige gesättigte oder ungesättigte Alkylengruppe mit bis zu 10 C-Atomen, die gegebenenfalls durch Fluor substituiert sein kann, oder die Gruppe 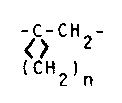 mit n=1-3;
- D: ist eine Direktbindung, Sauerstoff, Schwefel, eine -C≡-C-Gruppe oder eine -CH=CR⁷-Gruppe mit R⁷ als Wasserstoff, C₁₋₅-Alkyl, Chlor, Brom oder 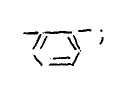
- B und D: sind gemeinsam, eine Direktbindung;
- R² und R³: sind gleich oder verschieden und bedeuten Wasserstoff oder einen organischen Säurerest mit 1-15 C-Atomen;
- R¹ und R²: sind gemeinsam eine Carbonylgruppe;
- R⁴: ist ein Wasserstoffatom, C₁₋₁₀-Alkyl, Cycloalkyl mit 5-6 C-Atomen , ein gegebenenfalls durch 1-2 Chlor, Brom, Phenyl, 6₁₋₄-Alkyl, C₁₋₄-Alkoxy, Chlormethyl, Fluormethyl, Trifluormethyl, Carboxy oder Hydroxy substituierter Phenylrest und falls
- R⁵: ein Wasserstoffatom bedeutet, deren Salze mit physiologisch verträglichen Basen und deren Cyclodextrinclathrate.

Besonders bevorzugte Verbindungen der vorliegenden Erfindung sind Verbindungen der Formel I, wobei die Reste die folgende Bedeutung haben: 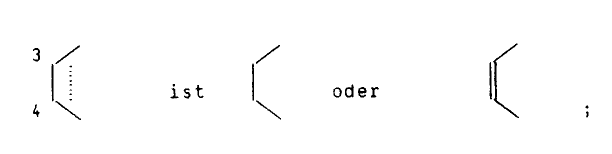
- R¹: ist CH₂OH, COOR⁵ mit R⁵ in der Bedeutung eines Wasserstoffatoms, eines Alkylrestes mit 1-4 C-Atomen;
- A: ist eine trans, trans-CH=CH-CH=CH- oder Tetramethylengruppe;
- B: ist eine geradkettige oder verzweigtkettige Alkylengruppe mit bis zu 5 C-Atomen;
- D: ist eine Direktbindung oder eine -C≡C-Gruppe oder eine -CH=CR⁷ -Gruppe mit R⁷ als Wasserstoff oder C₁₋₅-Alkyl; oder 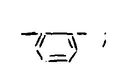
- B und D: sind gemeinsam eine Direktbindung;
- R² und R³: sind gleich oder verschieden und bedeuten Wasserstoff oder einen organischen Säurerest mit 1-6 C-Atomen;
- R¹ und R²: sind gemeinsam eine Carbonylgruppe;
- R⁴: ist ein Wasserstoffatom oder C₁₋₁₀-Alkyl und falls R⁵ ein Wasserstoffatom bedeutet, deren Salze mit physiologisch verträglichen Basen und deren Cyclodextrinclathrate.

Außerdem betrifft die Erfindung ein Verfahren zur Herstellung der erfindungsgemäßen Verbindungen der Formel I , das dadurch gekennzeichnet ist, daß man einen Aldehyd der Formel II 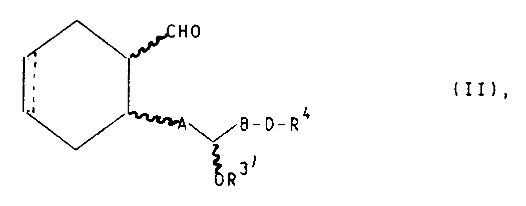
worin A, B, D und R⁴ die oben angegebene Bedeutung haben und R^{3'} Silylschutzgruppen bedeutet, wie sie für R⁸ genannt sind, gegebenenfalls nach Schutz freier Hydroxygruppen mit einer magnesiumorganischen Verbindung der Formel III,

X-Mg-CH₂-CH₂-CH₂-CH₂-O-R⁸ (III),

worin X Chlor, Brom oder Jod und R⁸ einen leicht abspaltbaren Etherrest bedeuten, umsetzt und gegebenenfalls anschließend in beliebiger Reihenfolge Isomere trennt, geschützte Hydroxygruppen freisetzt und/oder eine freie Hydroxygruppe verestert und/oder die 1-Hydroxygruppe zur Carbonsäure oxidiert und/oder Doppelbindungen hydriert und/oder eine veresterte Carboxylgruppe (R¹=COOR⁵) verseift und/oder reduziert und/oder eine Carboxylgruppe (R⁵=H) verestert und/oder eine freie Carboxylgruppe (R⁵=H) in ein Amid (R¹=CONHR⁶) überführt oder eine Carboxylgruppe mit einer physiologisch verträglichen Base in ein Salz überführt.

Als Etherrester R⁸ und R^{3'} in der Verbindung der Formeln II und III kommen die dem Fachmann geläufigen Reste in Betracht. Bevorzugt sind leicht abspaltbare Etherreste, wie beispielsweise Dimethyl-tert.-butylsilyl, Trimethylsilyl-, Tribenzylsilyl-, Diphenyl-tert.-butylsilyl-, Tetrahydropyranyl-, Tetrahydrofuranyl- und α-Ethoxyethyl, um nur einige zu nennen.

Die Umsetzung der Verbindung der Formel II mit einer metallorganischen Verbindung der Formel III erfolgt in an sich bekannter Weise in einem inerten Lösungsmittel oder Lösungsmittelgemisch, wie zum Beispiel Diethylether, Tetrahydrofuran, Dioxan, Toluol, Dimethoxyethan, vorzugsweise Diethylether oder Tetrahydrofuran. Die Reaktion wird bei Temperaturen zwischen -100 °C und 60 °C, vorzugsweise bei -78 °C bis 0 °C durchgeführt.

Die Herstellung der für diese Umsetzung benötigten Verbindung der Formel III erfolgt durch Reaktion des entsprechenden Hydroxyhalogenids durch Veretherung mit Dihydropyran und p-Toluolsulfonsäure und anschließende Umsetzung mit Magnesium.

Die Reduktion zu den Verbindungen der Formel I mit R¹ in der Bedeutung einer CH₂OH-Gruppe wird mit einem für die Reduktion von Estern oder Carbonsäuren geeigneten Reduktionsmittel wie beispielsweise Lithiumaluminiumhydrid, Diisobutylaluminiumhydrid usw. durchgeführt. Als Lösungsmittel kommen Diethylether, Tetrahydrofuran, Dimethoxyethan, Toluol usw. in Frage. Die Reduktion wird bei Temperaturen von -30 °C bis zur Siedetemperatur des verwendeteten Lösungsmittels, vorzugsweise 0 °C bis 30 °C vorgenommen.

Die Veresterung der Alkohole der Formel I (R² = H und/oder R³ = H) erfolgt in an sich bekannter Weise. Zum Beispiel erfolgt die Veresterung dadurch, daß man ein Säurederivat, vorzugsweise ein Säurehalogenid oder Säureanhydrid, in Gegenwart einer Base wie beispielsweise NaH, Pyridin, Triethylamin, Tributylamin oder 4-Dimethylaminopyridin mit einem Alkohol der Formel I umsetzt. Die Umsetzung kann ohne Lösungsmittel oder in einem inerten Lösungsmittel vorzugsweise Aceton, Acetonitril, Dimethylacetamid, DMSO bei Temperaturen über oder unter Raumtemperatur, zum Beispiel zwischen -80 °C bis 100 °C, vorzugsweise bei Raumtemperatur, vorgenommen werden.

Die Oxidation der l-Hydroxygruppe wird nach den dem Fachmann bekannte Methoden vorgenommen. Als Oxidationsmittel können beispielsweise dienen: Pyridiniumdichromat (Tetrahedron Letters, 1979, 399), Jones-Reagenz (J. Chem. Soc. 1953, 2555) oder Platin/Sauerstoff (Adv. in Carbohydrate Chem. 17, 169 (1962) oder Collins-Oxidation und anschließende Jones-Oxidation. Die Oxidation mit Pyridiniumdichromat wird bei Temperaturen von 0 °C bis 100 °C, vorzugsweise 20 °C bis 40 °C in einem gegen das Oxidationsmittel inerten Lösungsmittel, beispielsweise Dimethylformamid, durchgeführt.

Die Oxidation mit Jones-Reagenz wird bei Temperaturen von -40 °C bis +40 °C, vorzugsweise 0 °C bis 30 °C in Aceton als Lösungsmittel ausgeführt.

Die Oxidation mit Platin/Sauerstoff wird bei Temperaturen von 0 °C bis 60 °C, vorzugsweise 20 °C bis 40 °C in einem gegen das Oxidationsmittel inerten Lösungsmittel, wie z. B. Essigester, durchgeführt.

Die Verseifung der Ester der Formel I wird nach den dem Fachmann bekannten Methoden durchgeführt, wie beispielsweise mit basischen Katalysatoren. Die Verbindungen der Formel I können durch die üblichen Trennmethoden in die optischen Isomeren getrennt werden.

Die Freisetzung der funktionell abgewandelten Hydroxygruppen erfolgt nach bekannten Methoden. Beispielweise wird die Abspaltung von Hydroxyschutzgruppen, wie beispielsweise des Tetrahydropyranylrestes, in einer wässrigen Lösung einer organischen Säure, wie z. B. Oxalsäure, Essigsäure, Propionsäure u. a., oder in einer wässrigen Lösung einer anorganischen Säure, wie z. B. Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind z. B. Alkohole, wie Methanol und Ethanol, und Ether, wie Dimethoxyethan, Dioxan und Tetrahydrofuran. Tetrahydrofuran wird bevorzugt angewendet. Die Abspaltung wird vorzugsweise bei Temperaturen zwischen 20 °C und 80 °C durchgeführt. Die Abspaltung der Silyletherschutzgruppen erfolgt beispielsweise mit Tetrabutylammoniumfluorid oder mit Kaliumfluorid in Gegenwart eines Kronenethers. Als Lösungsmittel sind beispielsweise geeignet Tetrahydrofuran, Diethylether, Dioxan, Methylenchlorid usw. Die Abspaltung wird vorzugsweise bei Temperaturen zwischen 0 °C und 80 °C durchgeführt.

Die Verseifung der Acylgruppen erfolgt beispielsweise mit Alkali- oder Erdalkali-carbonaten oder -hydroxyden in einem Alkohol oder in der wässrigen Lösung eines Alkohols. Als Alkohol kommen aliphatische Alkohole in Betracht, wie z.B. Methanol, Ethanol, Butanol usw., vorzugsweise Methanol. Als Alkalicarbonate und -hydroxyde seien Kalium- und Natriumsalze genannt. Bevorzugt sind die Kaliumsalze.

Als Erdkalicarbonate und -hydroxide sind beispielsweise geeignet Calciumcarbonat, Calciumhydroxyd und Bariumcarbonat. Die Umsetzung erfolgt bei -10 °C bis +70 °C, vorzugsweise bei +25 °C.

Die Einführung der Estergruppe 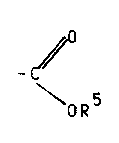
für R¹, bei welcher R⁵ eine Alkylgruppe mit 1-10 C-Atomen darstellt, erfolgt nach den dem Fachmann bekannten Methoden. Die l-Carboxy-verbindungen werden beispielsweise mit Diazokohlenwasserstoffen in an sich bekannter Weise umgesetzt. Die Veresterung mit Diazokohlenwasserstoffen erfolgt z. B. dadurch, daß man eine Lösung des Diazokohlenwasserstoffes in einem inerten Lösungsmittel, vorzugsweise in Diethylether, mit der 1-Carboxyverbindung in dem gleichen oder in einem anderen inerten Lösungsmittel, wie z. B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389 - 394 (1954)].

Die Einführung der Estergruppe 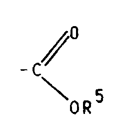
für R¹, bei welcher R² eine substituierte oder unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base, beispielsweise Pyridin, DMAP, Triethylamin, in einem inerten Lösungsmittel umgesetzt. Als Lösungsmittel kommen Methylenchlorid, Ethylenchlorid, Chloroform, Essigester, Tetrahydrofuran, vorzugsweise Chloroform in Frage. Die Reaktion wird bei Temperaturen zwischen -30 °C und +50 °C, vorzugsweise bei 10 °C, durchgeführt.

Sollen im Primärprodukt enthaltene C=C-Doppelbindungen reduziert werden, erfolgt die Hydrierung nach an sich bekannten Methoden.

Die Hydrierung des Δ^{8,10}-Diensystems wird in an sich bekannter Weise bei tiefen Temperaturen, vorzugsweise bei etwa -20 °C bis +30 °C in einer Wasserstoffatmosphäre in Gegenwart eines Edelmetallkatalysators durchgeführt. Als Katalysator ist zum Beispiel 10 % Palladium auf Kohle geeignet.

Die Leukotrien-B₄-Derivate der Formel I mit R¹ in der Bedeutung einer COOH-Gruppe können mit geeigneten Mengen der entsprechenden anorganischen Basen unter Neutralisierung in ein Salz überführt werden. Beispielsweise erhalt man beim Lösen der entsprechenden Säuren in Wasser, das die stöchiometrische Menge der Base enthält, nach Abdampfen des Wassers oder nach Zugabe eines mit Wasser mischbaren Lösungsmittels, z.B. Alkohol oder Aceton, das feste anorganische Salz.

Zur Herstellung eines Aminsalzes wird die LTB₄ -Säure z.B. in einem geeigneten Lösungsmittel, beispielsweise Ethanol, Aceton, Diethylether, Acetonitril oder Benzol gelöst und mindestens die stöchiometrische Menge des Amins dieser Lösung zugesetzt. Dabei fällt das Salz gewöhnlich in fester Form an oder wird nach Verdampfen des Losungsmittels in üblicher Weise isoliert.

Die Einführung der Amidgruppe 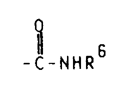
für R¹ erfolgt nach den dem Fachmann bekannten Methoden. Die Carbonsäuren der Formel I (R⁵=H), werden zunächst in Gegenwart eines tertiären Amins, wie beispielsweise Triethylamin, mit Chlorameisensäureisobutylester in das gemischte Anhydrid überführt. Die Umsetzung des gemischten Anhydrids mit dem Alkalisalz des entsprechenden Amids oder mit Ammoniak (R⁶=H) erfolgt in einem inerten Lösungsmittel oder Lösungsmittelgemisch, wie beispielsweise Tetrahydrofuran, Dimethoxyethan, Dimethylformamid, Hexamethylphosphorsäuretriamid, bei Temperaturen zwischen -30 °C und +60 °C, vorzugsweise bei 0 °C bis 30 °C.

Eine weitere Möglichkeit für die Einführung der Amidgruppe 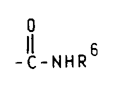
für R¹ besteht in der Umsetzung einer 1-Carbonsäure der Formel I (R⁵=H), in der freie Hydroxygruppen gegebenenfalls intermediär geschützt sind, mit Verbindungen der Formel IV,

O = C = N - R⁶ (IV),

worin R⁶ die oben angegebene Bedeutung hat.

Die Umsetztung der Verbindung der Formel I (R⁵=H) mit einem Isocyanat der Formel IV erfolgt gegebenenfalls unter Zusatz eines tertiären Amins, wie z.B. Triethylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diethylether, Toluol, bei Temperaturen zwischen -80 °C bis 100 °C, vorzugsweise bei 0 °C bis 30 °C, vorgenommen werden.

Enthält das Ausgangsprodukt OH-Gruppen im Leukotrien-B₄-Rest so werden diese OH-Gruppen auch zur Reaktion gebracht. Werden letztlich Endprodukte gewünscht, die freie Hydroxylgruppen enthalten, geht man zweckmässigerweise von Ausgangsprodukten aus, in denen diese durch vorzugsweise leicht abspaltbare Ether- oder Acylreste intermediär geschützt sind.

Die Trennung der Enantiomeren und/oder Diastereomeren erfolgt nach den dem Fachmann bekannten Methoden, z.B. durch chromatographische Methoden, beispielsweise Hochdruckflüssigchromatographie an optisch aktiven Trägermaterialien.

Die als Ausgangsmaterial dienenden Verbindungen der Formel II können beispielsweise hergestellt werden, indem man in an sich bekannter Weise 2-(Hydroxymethyl)-benzylalkohol in den Monosilylether der Formel V überführt. 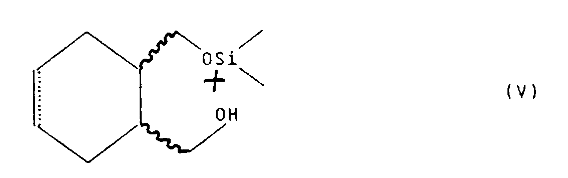
Durch Oxidation z.B. mit Collins-Reagenz oder durch das Swern-Verfahren wird der Aldehyd der Formel VI erhalten, 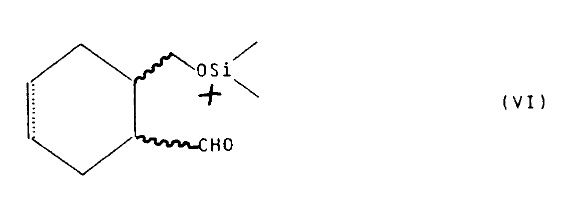 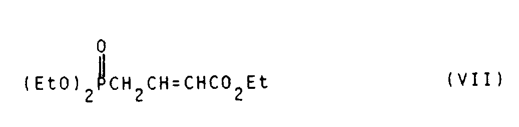
der in einer Wittig-Horner-Olefinierung mit dem Phosphonat der Formel VII und einer Base und gegebenenfalls anschließender Hydrierung in den Ester der Formel VIII überführt wird, wobei A die 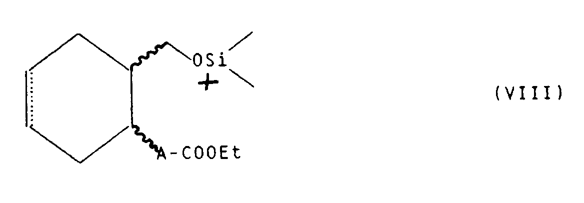
oben angegebene Bedeutung besitzt. Als Basen kommen beispielsweise Kalium-tert.-butylat, Diazabicyclononan oder Natriumhydrid infrage. Reduktion der Estergruppe, beispielsweise mit DIBAH und anschließende Oxidation des erhaltenen primären Alkohols z.B. mit Braunstein oder Collinsreagenz führt zum Aldehyd der Formel IX 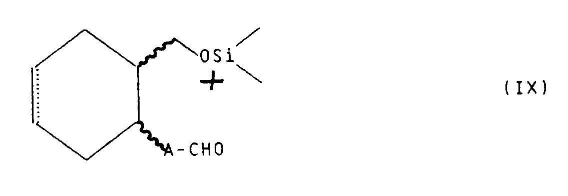
Die metallorganische Umsetzung des Aldehyds der Formel IX mit einem Grignardreagenz der Formel X, worin B, D

X-Mg-B-D-R⁴ (X)

und R⁴ die oben angegebenen Bedeutungen aufweisen und X Chlor, Brom oder Jod bedeutet, führt nach Schutz der Hydroxygruppe und gegebenenfalls Diastereomerentrennung (beispielsweise durch Acylierung) zu den Verbindungen der Formel XI 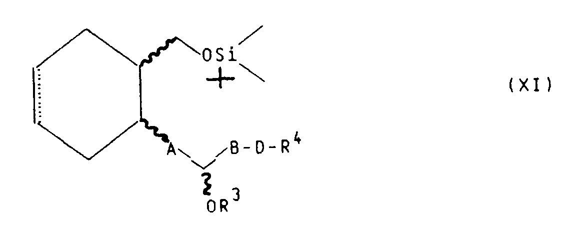
Die Herstellung der für die metallorganische Umsetzung benötigten Verbindung der Formel X erfolgt durch Reaktion des entsprechenden terminalen Halogenids mit Magnesium. Durch Umsetzung des Silylethers XI mit Tetrabutylammoniumfluorid wird der Alkohol der Formel XII erhalten. 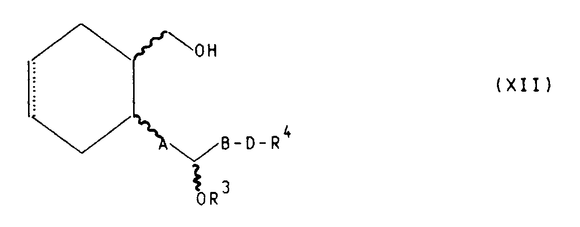
Die Oxidation der primären Alkoholgruppe in XII z. B. mit Collinsreagenz oder Pyridiniumdichromat führt zum Aldehyd der Formel II.

Zu den Verbindungen der Formel XI, worin B eine CH₂-Gruppe und D eine -C≡C-oder CH=CR⁷-Gruppe bedeutet, kann man gelangen, beispielsweise durch eine metallorganische Umsetzung eines Propargylhalogenids mit dem Aldehyd der Formel IX und anschließende Alkylierung mit einem entsprechenden Alkylhalogenid und gegebenenfalls anschließende Lindlar-Hydrierung. Die Verbindungen der Formel I wirken antientzündlich und antiallergisch. Daneben besitzen sie antimykotische Eigenschaften. Folglich stellen die neuen Leukotrien B₄-Derivate der Formel I wertvolle pharmazeutische Wirkstoffe dar. Die Verbindungen der Formel I sind besonders zur topischen Applikation geeignet, da sie eine Dissoziation zwischen erwünschter topischer Wirksamkeit und unerwünschten systemischen Nebenwirkungen aufweisen.

Die neuen Leukotrien B₄-Derivate der Formel I eignen sich in Kombination mit den in der galenischen Pharmazie üblichen Hilfs- und Trägermitteln zur lokalen Behandlung von Kontaktdermatitis, Ekzemen der verschiedensten Art, Neurodermatosen, Erythrodermie, Verbrennungen, Tinea, Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigeneten Zusätzen in die gewünschte Applikationsform, wie zum Beispiel: Lösungen, Lotionen, Salben, Cremes oder Pflaster, überführt.

In den so formulierten Arzneimitteln ist die Wirkstoffkonzentration von der Applikationsform abhängig. Bei Lotionen und Salben wird vorzugsweise eine Wirkstoffkonzentration von 0,0001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Leukotrien-B₄-Derivate auch in Form von Kapseln, Tabletten oder Dragees, die vorzugsweise o,1 bis 100 mg Wirkstoff enthalten oder oral appliziert werden oder in Form von Suspensionen, die vorzugsweise 1-200 mg Wirkstoff pro Dosiseinheit enthalten und rektal appliziert werden auch zur Behandlung allergischer Erkrankungen des Darmtraktes, wie der Kolitis ulcerosa und der colitis granulomatosa.

Die neuen Leukotrien-B₄-Derivate können auch in Kombination, wie z.B mit Lipoxygenasehemmern, Cyclooxygenasehemmern, Prostacyclinagonisten Thromboxanantagonisten, Leukotrien D₄-antagonisten, Leukotrien E₄-antagonisten, Leukotrien F₄-antagonisten, Phosphodiesterasehemmern, Calciumantagonisten oder PAF-Antagonisten verwendet werden.

### Beispiel 1

### (+)-(5RS)-5-Acetoxy-5-[cis-(6RS)-6-((1E,3E)-(5RS)-5-acetoxy-1,3-tridecadienyl)-(1RS)-3-cyclohexen-1-yl]-pentanol unpolares und polares Diastereomeres.

Zu 4 g Magnesium tropft man bei 25°C unter Argon eine Lösung aus 16 g 4-Chlor-1-(tert.-butyldimethylsilyloxy)-butan in 14,4 ml Tetrahydrofuran und 0,84 ml Dibrommethan, erwärmt 10 Minuten auf 70°C, rührt 30 Minuten bei 25°C und verdünnt mit 45,2 ml Tetrahydrofuran.
Zu 29,9 ml dieser magnesiumorganischen Lösung tropft man bei -20°C unter Argon eine Lösung aus 4,2 g cis-(1RS)-1-Formyl-(6RS)-6-[(1E,3E)-(5RS)-5-acetoxy-1,3-tridecadienyl]-3-cyclohexen in 8,25 ml Tetrahydrofuran und rührt 30 Minuten bei -20°C. Man versetzt mit 150 ml gesättigter Ammoniumchloridlösung, extrahiert dreimal mit Ether, schüttelt die organische Phase mit Sole, trocknet über Magnesiumsulfat und dampft im Vakuum ein. Den Rückstand chromatographiert man an Kieselgel. Mit Hexan/Essigester (8+2) erhält man 4,1 g des Alkohols als farbloses Öl.
- IR (CHCl₃):: 3600, 2930, 2858, 1725, 1250, 992, 838 cm⁻¹.

Zur Acetylierung fügt man zu einer Lösung aus 6 g des vorstehend beschriebenen Alkohols in 38 ml Pyridin 6,3 ml Essigsäureanhydrid und rührt 16 Stunden bei 24°C. Anschließend engt man im Vakuum unter Zusatz von Toluol ein und chromatographiert den Rückstand an Kieselgel. Mit Hexan/Essigester (95+5) erhält man 4,9 g des Acetats als farbloses Öl.
- IR:: 2930, 2860, 1728, 1255, 991, 838 cm⁻¹.

Zur Schutzgruppenabspaltung rührt man 4,55 g des vorstehend hergestellten Acetats in 320 ml Tetrahyrofuran mit 7,5 g Tetrabutylammoniumfluorid 1 Stunde bei 0°C und 4 Stunden bei 24°C unter Argon. Anschließend verdünnt man mit Äther, wäscht dreimal mit Wasser, trocknet über Magnesiumsulfat und dampft im Vakuum ein. Den Rückstand chromatographiert man mit Hexan/Essigester (6+4) an Kieselgel. Dabei erhält man zunächst 1,32 g des unpolaren Diastereomeren und dann 1,7 g des polareren Diastereomeren der Titelverbindung als farblose Öle.
- IR (unpolares Diastereomeres):: 3630, 3430, 2930, 2860, 1728, 1608, 1375, 1250, 992 cm⁻¹.
- IR (polares Diastereomeres):: 3630, 3480, 2930, 2860, 1729, 1608, 1375, 1250, 992 cm⁻¹.

Das Ausgangsmaterial für die obige Titelverbindung wird wie folgt hergestellt:
1a) 5-cis-1-(tert.-butyl-dimethylsilyloxymethyl)-3-cyclohexen-6-yl]-(2E,4E)-pentadiensäureäthylester
   Zu einer Suspension von 10 g Lithiumaluminiumhydrid in 140 ml Tetrahydrofuran tropft man bei Raumtemperatur eine Lösung von 25 g cis-4-cyclohexen-1,2-dicarbonsäureanhydrid in 140 ml Tetrahydrofuran und rührtt die Mischung anschließend 3 Stunden bei Rückflußtemperatur. Man kühlt auf 0°C, tropft langsam eine Mischung aus Tetrahydrofuran/Wasser (1+1) zu, rührt 30 Minuten, versetzt mit 150 ml Chloroform, filtriert und dampft das Filtrat im Vakuum ein. Den Rückstand destilliert man bei 0,2 mm/ (Torr und 150°C am Kugelrohr. Nach Umkristallisation des Destillats aus Toluol/Pentan erhält man 21 g cis-1,6-Dihydroxymethyl-3-cyclohexen als farblose Kristalle, Fp. 33-35°C.
   Zu einer Lösung von 17,7 g des vorstehend hergestellten Diols und 17,2 g Imidazol in 147 ml Dimethylformamid fügt man bei 0°C unter Argon 19 g tert.-Butyldimethylsilylchlorid und rührt 16 Stunden bei 25°C. Man verdünnt mit 1,5 ltr. Äther, schüttelt zweimal mit je 100 ml 10%iger Schwefelsäure, wäscht mit Sole neutral, trocknet über Magnesiumsulfat und dampft im Vakuum ein. Den Rückstand chromatographiert man an Kieselgel. Mit Hexan/Essigester (9+1) erhält man 16,2 g cis-1-(tert.-Butyl-dimethylsilyloxymethyl)-6-hydroxymethyl)-3-cyclohexen als farbloses Öl.
   - IR:: 3580, 3390, 2930, 2858, 835 cm⁻¹.
   Zu einer Lösung von 14 g des vorstehend beschriebenen Monosilyläthers in 700 ml Methylenchlorld fügt man bei 0°C 140 g Collins-Reagenz (Chromsäure-Pyridin-Komplex) und rührt 40 Minuten bei 0°C. Anschließend verdünnt man mit einer Mischung aus Hexan/Äther (3+2), fügt Celite hinzu, filtriert und dampft im Vakuum ein. Den Rückstand chromatographiert man mit Hexan/Essigester (9+1) an Kieselgel. Dabei erhält man 12,8 g cis-1-(tert.-Butyl-dimethylsilyloxymethyl)-6-formyl-3-cyclohexen als farbloses Öl.
   - IR:: 2930, 2860, 2730, 1715, 838 cm⁻¹.
   Zur Wittig-Horner-Olefinierung fügt man bei 24°C 12,4 g Phosphonocrotonsäuretriäthylester und 6,3 g Diazabicycloundecen (BDU) zu einer gerührten Suspension von 2,1 g Lithiumchlorid in 412 ml Acetonitril und rührt 10 Minuten. Anschließend tropft man eine Lösung aus 10,5 g des vorstehend beschriebenen Aldehyds in 83 ml Acetonitril zu, rührt 3 Stunden bei 24°C und verdünnt dann mit Äther. Man schüttelt nacheinander mit Wasser, 10%iger Citronensäurelösung und Wasser, trocknet über Magnesiumsulfat und dampft im Vakuum ein. Den Rückstand chromatographiert man mit Hexan/Essigester (95+5) an Kieselgel. Dabei erhält man 9 g der Titelverbindung als farbloses Öl.
   - IR:: 2930, 2858, 1710, 1638, 1618, 1256, 1003, 940,838 cm⁻¹.
1b) 5-[cis-1-(tert.-Butyl-dimethylsilyloxymethyl)-3-cyclohexen-6-yl]-(2E,4E)-pentadien-1-al
   Zu einer Lösung von 8,8 g des nach Beispiel 1a hergestellten Esters in 200 ml Toluol tropft man bei -70°C unter Argon 41 ml einer 1,2 molaren Lösung von Diisobutylaluminiumhydrid ion Toluol und rührt 40 Minuten bei -70°C. Anschließend tropft man 4 ml Isopropanol und dann 21 ml Wasser zu, rührt 2 Stunden bei 22°C, filtriert, wäscht mit Methylenchlorid und dampft im Vakuum ein. Den Rückstand reinigt man durch Chromatographie an Kieselgel. Mit Hexan/Essigester (8+2) erhält man 7,1 g des Alkohols als farbloses Öl.
   - IR:: 3630, 3460, 838 cm⁻¹.
   Eine Lösung von 7,1 g des vorstehend hergestellten Alkohols in 260 ml Methylenchlorid versetzt man mit 20 g Braunstein und rührt 4 Stunden bei 24°C. Anschließend wird filtriert, eingedampft und an Kieselgel chromatographiert. Mit Hexan/Essigester (85+15) eluiert man 6,2 g der Titelverbindung als farbloses Öl.
   - IR:: 2930, 2860, 1680, 1638, 990, 950, 838 cm⁻¹.
1c) (5RS)-5-Acetoxy-1-[cis-1-(tert.-Butyl-dimethylsilyloxymethyl)-3-cyclohexen-6-yl]-(1E,3E)-tri-decadien
   Zu 1,2 g Magnesium in 5 ml Ether tropft man unter Erwärmen eine Lösung aus 7,95 ml Octylbromid in 12 ml Ether und rührt 30 Minuten bei 25°C. Zu 5,85 ml (^10,77 mMol) dieser Grignardlösung tropft man bei -20°C unter Argon eine Losung aus 3,0 g des nach Beispiel 1b hergestellten Aldehyds in 48 ml Ether und rührt 3 Stunden bei -20°C. Man versetzt mit gesättigter Ammoniumchloridlösung, extrahiert dreimal mit Ether, schüttelt die organische Phase mit Sole, trocknet über Magnesiumsulfat und dampft im Vakuum ein. Den Rückstand chromatographiert man an Kieselgel. Mit Hexan/Essigester (7+3) eluiert man 3,35 g des Alkohols als farbloses Öl. IR: 3620, 3460, 2930, 2858, 992, 838.
   Zur Acetylierung fügt man zu einer Lösung von 3,3 g des vorstehend hergestellten Alkohols in 12 ml Pyridin 6 ml Essigsäureanhydrid und rührt 16 Stunden bei 24°C. Anschließend engt man im Vakuum unter Zusatz von Toluol ein und chromatographiert den Rückstand an Kieselgel. Mit Hexan/Essigester (8+21) erhält man 3,3 g der Titelverbindung als Öl. IR: 2930, 2858, 1730, 1254, 990, 838 cm⁻¹.
1c) (5RS)-5-Acetoxy-1-[cis-1-(tert.-Butyl-dimethylsilyloxymethyl)-3-cyclohexen-6-yl]-(1E,3E)-tridecadien
   Zu 1,2 g Magnesium in 5 ml Äther tropft man unter Erwärmen eine Lösung aus 7,95 ml Octylbromid in 12 ml Äther und rührt 30 Minuten bei 25 °C.
   Zu 5,85 ml (= 10,77 mMol) dieser Grignard-Lösung tropft man bei -20 °C unter Argon eine Lösung aus 3,0 g des nach Beispiel 16 hergestellten Aldehyds in 48 ml Äther und rührt 3 Stunden bei -20 °C. Man versetzt mit gesättigter Ammoniumchloridlösung, extrahiert dreimal mit Äther, schüttelt die organische Phase mit Sole, trocknet über Magnesiumsulfat und dampft im Vakuum ein. Den Rückstand chromatographiert man an Kieselgel. Mit Hexan/Essigester (7+3) eluiert man 3,35 g des Alkohols als farbloses Öl.
   - IR:: 3620, 3460, 2930, 2858, 992, 838.
   Zur Acetylierung fügt man zu einer Lösung von 3,3 g des vorstehend hergestellten Alkohols in 12 ml Pyridin 6 ml Essigsäureanhydrid und rührt 16 Stunden bei 24 °C. Anschließend engt man im Vakuum unter Zusatz von Toluol ein und chromatographiert den Rückstand an Kieselgel. Mit Hexan/Essigester (8+2) erhält man 3,3 g der Titelverbindung als Öl.
   - IR:: 2930, 2858, 1730, 1254, 990, 838 cm⁻¹
1d)cis-(1RS)-1-Formyl-(6RS)-6-[(1E,3E)-(5RS)-5-acetoxy-1,3-tridecadienyl]-3-cyclohexen
   Zu einer Lösung von 3,2 g des nach Beispiel 1c) hergestellten Acetats in 320 ml Tetrahydrofuran fügt man bei 0 °C 6,55 g Tetrabutylammoniumfluorid, rührt 1 Stunde bei 0 °C und 3 Stunden bei 24 °C. Anschließend verdünnt man mit 1,2 l Äther und wäscht dreimal mit Sole. Man trocknet über Magnesiumsulfat, dampft im Vakuum ein und chromatographiert den Rückstand an Kieselgel. Mit Hexan/Essigester (6+4) eluiert man 2,4 g des Alkohols als farbloses Öl.
   - IR:: 3620, 3460, 2930, 2860, 1722, 1250, 991 cm⁻¹.

   Zu einer Lösung von 2,71 g des vorstehend hergestellten Alkohols in 75 ml Methylenchlorid fügt man bei 0 °C 19 g Collins-Reagenz (Chromsäure-Pyridin-Komplex) und rührt 15 Minuten bei 0 °C. Anschließend verdünnt man mit einer Mischung aus Hexan/Äther (3+2), fügt Celite hinzu, filtriert und dampft im Vakuum ein. Der so erhaltene Aldehyd wird ohne weitere Reinigung verwendet.

### Beispiel 2

### (+)-(5RS)-5-Acetoxy-5-[cis-(6RS)-6-(1E,3E)-(5RS)-5-acetoxy-1,3-tridecadienyl)-(1RS)-3-cyclohexen-1-yl]-pentansäure Diastereomeres A

Zu einer Lösung von 350 mg des in Beispiel 1 beschriebenen unpolaren diastereomeren Diacetats in 28 ml Methylenchlorid fügt man bei 0 °C 1,92 g Collins-Reagenz und rührt 15 Minuten bei 0 °C. Anschließend verdünnt man mit einer Mischung aus Hexan/Äther (3+2), fügt Celite hinzu, filtriert, wäscht mit Hexan/Äther (3+2) und dampft im Vakuum ein.

Zu einer Lösung von 360 mg des vorstehend hergestellten Aldehyds in 12,5 ml Aceton tropft man unter Rühren bei -20 °C 0,7 ml Jones-Reagenz (J. Chem. Soc. 1953, 2555) und rührt 12 Minuten bei -20 °C unter Argon. Anschließend fügt man 3 ml Isopropanol zu, rührt 10 Minuten, verdünnt mit 40 ml Ether, filtriert, wäscht mit Ether, schüttelt die Etherphase zweimal mit Sole, trocknet über Magnesiumsulfat und dampft im Vakuum ein. Den Rückstand chromatographiert man an Kieselgel. Mit Hexan/Essigester (6+4) erhält man 290 mg der Titelverbindung als farbloses Öl.
- IR:: 3520 (breit), 2928, 2859, 1725, 1372, 1250, 991, 948 cm⁻¹.

### Beispiel 3

### (+)-(RS)-5-Hydroxy-5-[cis-(6RS)-6-((1E,3E)-(5RS)-5-hydroxy-1,3-tridecadienyl)-(1RS)-3-cyclohexen-1-yl]-pentansäure Diastereomeres A

60 mg des in Beispiel 2 beschriebenen unpolaren diasteremoren Diacetats (Diastereomeres A) rührt man 48 Stunden bei 24 °C mit 2,5 ml einer Lösung von Kaliumhydroxid in Wasser und Äthanol (Herstellung: man löst 5 g Kaliumhydroxid in 67,5 ml Wasser und 182,5 ml Äthanol). Anschließend säuert man mit 10 %iger Zitronensäurelösung auf pH4 an, extrahiert viermal mit je 15 ml Methylenchlorid, schüttelt die organische Phase mit Sole, trocknet über Magnesiumsulfat und dampft im Vakuum ein. Den Rückstand chromatographiert man mit Essigester/Hexan (8+2) an Kieselgel. Dabei erhält man 41 mg der Titelverbindung als farbloses Öl.
- IR:: 3600, 3400 (breit), 2930, 2859, 1720, 1375, 992 cm⁻¹.

### Beispiel 4

### (+)-(5RS)-5-Acetoxy-5-[cis-(6RS)-6-((1E,3E)-(5RS)-5-hydroxy-1,3-tridecadienyl)-(1RS)-3-cyclohexen-1-yl]-pentansäure Diastereomeres A

Zu einer Lösung von 70 mg des nach Beispiel 2 hergestellten unpolaren diastereomeren Diacetats (Diastereomeres A) in 1,5 ml Methanol fügt man bei 24 °C 1,5 ml einer 0,5 normalen Natronlauge und rührt 7 Stunden bei 24 °C unter Argon. Anschließend verdünnt man mit 2 ml Wasser und säuert bei Eisbadtemperatur mit 10 %iger Zitronensäurelösung auf pH4 an. Man extrahiert viermal mit je 30 ml Essigester, schüttelt die organische Phase mit Sole, trocknet über Magnesiumsulfat und dampft im Vakuum ein. Den Rückstand chromatographiert man an Kieselgel. Mit Äther/Hexan (1+1) erhält man 51 mg der Titelverbindung als farbloses Öl.
- IR:: 3600, 3450, 2930, 2859, 1722, 1375, 1250, 992 cm⁻¹.

### Beispiel 5

### (+)-(5RS)-5-Hydroxy-5-[cis-(6RS)-6-((1E,3E)-(5RS)-5-hydroxy-1,3-tridecadienyl)-(1RS)-3-cyclohexen-1-yl]-pentanol Diastereomeres A

88 mg des in Beispiel 1 beschriebenen unpolaren diastereomeren Diacetats rührt man 60 Stunden bei 24 °C mit 2,7 ml einer Lösung von Kaliumhydroxid in Wasser und Äthanol (Herstellung: man löst 5 g Kaliumhydroxid in 67,5 ml Wasser und 182,5 ml Äthanol). Anschließend säuert man mit 10 %iger Zitronensäurelösung auf pH6 an, extrahiert viermal mit je 20 ml Methylenchlorid, schüttelt die organische Phase mit Sole, trocknet über Magnesiumsulfat und dampft im Vakuum ein. Den Rückstand chromatographiert man mit Essigester an Kieselgel. Dabei erhält man 48 mg der Titelverbindung als farbloses Öl.
- IR:: 3620, 3380 (breit), 2929, 2860, 992 cm⁻¹.

### Beispiel 6

### (+)-(5RS)-5-Acetoxy-5-[cis-(6RS)-6-((1E,3E)-(5RS)-5-hydroxy-1,3-tridecadienyl)-(1RS)-3-cyclohexen-1-yl]-pentanol Diasteremoeres A

58 mg des in Beispiel 1 beschriebenen unpolaren diasteremoeren Diacetats in 1,4 ml Methanol rührt man 4 Stunden bei 24 °C mit 1,4 ml einer 0,5 normalen Natronlauge unter Argon. Anschließend verdünnt man mit 5 ml Wasser, neutralisiert mit 10 %iger Zitronensäurelösung, extrahiert viermal mit je 20 ml Methylenchlorid, schüttelt die organische Phase mit Sole, trocknet über Magnesiumsulfat und dampft im Vakuum ein. Den Rückstand chromatographiert man mit Essig/Hexan (1+1) an Kieselgel. Dabei erhält man 21 mg der Titelverbindung als farbloses Öl.
- IR:: 3600, 3450 (breit), 2930, 2860, 1730, 1375, 1252, 993 cm⁻¹.

### Beispiel 7

### (+)-(5RS)-5-Acetoxy-5-[cis-(6RS)-6-((1E,3E)-(5RS)-5-Acetoxy-1,3-tridecadienyl)-(1RS)-3-cyclohexen-1-yl]-pentansäure Diastereomeres B

In Analogie zu Beispiel 2 erhält man aus 425 mg des nach Beispiel 1 hergestellten polaren diastereomeren Diacetats 315 mg der Titelverbindung als farbloses Öl.
- IR:: 3515 (breit), 2928, 2859, 1725, 1372, 1250, 991, 948 cm⁻¹.

### Beispiel 8

### (+)-(5RS)-5-Hydroxy-5-[cis-(6RS)-6-((1E,3E)-(5RS)-5-hydroxy-1,3-tridecadienyl)-(1RS)-3-cyclohexen-1-yl]-pentansäure Diastereomeres B

In Analogie zu Beispiel 3 erhält man aus 320 mg des nach Beispiel 7 hergestellten Diacetats (Diastereomeres B) 240 mg der Titelverbindung als farbloses Öl.
- IR:: 3600, 3420 (breit), 2930, 2860, 1720, 1375, 992 cm⁻¹.

### Beispiel 9

### (+)-(5RS)-5-Acetoxy-5-[cis-(6RS)-6-((1E,3E)-(5RS)-5-hydroxy-1,3-tridecadienyl)-(1RS)-3-cyclohexen-1-yl]-pentansäure Diastereomeres B

In Analogie zu Beispiel 4 erhält man aus 130 mg des nach Beispiel 7 hergestellten Diacetats (Diastereomeres B) 70 mg der Titelverbindung als farbloses Öl.
- IR:: 3620, 3400 (breit), 2930, 2859, 1722, 1375, 1250, 992 cm⁻¹.

### Beispiel 10

### (+)-(5RS)-5-Hydroxy-5-[cis-(6RS)-6-((1E,3E)-(5RS)-5-hydroxy-1,3-tridecadienyl)-(1RS)-3-cyclohexen-1-yl]-pentanol Diastereomeres B

In Analogie zu Beispiel 5 erhält man aus 145 mg des in Beispiel 1 hergestellten polaren diastereomeren Diacetats 81 mg der Titelverbindung als farbloses Öl.
- IR:: 3600, 3400 (breit), 2930, 2860, 992 cm⁻¹.

### Beispiel 11

### (+)-(5RS)-5-Acetoxy-5-[cis-(6RS)-6-((1E,3E)-(5RS)-5-hydroxy-1,3-tridecadienyl)-(1RS)-3-cyclohexen-1-yl]-pentanol Diastereomeres B

In Analogie zu Beispiel 6 erhält man aus 70 mg des in Beispiel 1 hergestellten polaren diastereomeren Diacetats 40 mg der Titelverbindung als farbloses Öl.
- IR:: 3620, 3430 (breit), 2930, 2860, 1730, 1375, 1250, 993 cm⁻¹.

### Beispiel 12

### (+)-(5RS)-5-Acetoxy-5-[cis-(2RS)-2-((1E,3E)-(5RS)-5-acetoxy-1,3-tridecadienyl)-(1RS)-cyclohex-1-yl]-pentanol unpolares und polares Diastereomeres

In Analogie zu Beispiel 1 erhält man aus 4 g cis-(1RS)-1-Formyl-(2RS)-2-[(1E,3E)-(5RS)-5-acetoxy-1,3-tridecadienyl]-cyclohexan 1,2 g des unpolareren Diastereomeren und 1,3 g des polareren Diastereomeren der Titelverbindung als farblose Öle.
- IR (unpolares Diastereomeres):: 3620, 3420, 2929, 2859, 1729, 1607, 1375, 1250, 992 cm⁻¹.
- IR (polares Diastereomeres):: 3600, 3430, 2929, 2860, 1729, 1607, 1375, 1250, 992 cm⁻¹.

Das Ausgangsmaterial für die obige Titelverbindung wird wie folgt hergestellt:
12a) 5-[cis-1-(tert.-Butyl-dimethylsilyloxymethyl)-cyclohex-2-yl]-(2E,4E)-pentadiensäureäthylester
   In Analogie zu Beispiel 1a) erhält man aus cis-1,2-Dihydroxymethylcyclohexan die Titelverbindung als farbloses Öl. IR: 2929, 2859, 1710, 1638, 1618, 1255, 1004, 940, 838 cm⁻¹.
12b) 5-[cis-1-(tert.-Butyl-dimethylsilyloxymethyl)-cyclohex-2-yl]-(2E,4E)-pentadien-1-al
   In Analogie zu Beispiel 1b) erhält man aus 16 g des nach Beispiel 12a) hergestellten Esters 13 g der Titelverbindung als farbloses Öl. IR: 2930, 2859, 1680, 1640, 992, 950, 840 cm⁻¹.
12c) (5RS)-5-Acetoxy-1-[cis-1-(tert.-Butyl-dimethyl-silyloxymethyl-cyclohex-1-yl]-(1E,3E)-tridecadien
   In Analogie zu Beispiel 1c) erhält man aus 4,2 g des nach Beispiel 12b) hergestellten Aldehyds 3,8 g der Titelverbindung als farbloses Öl. IR: 2928, 2859, 1730, 1255, 990, 838 cm⁻¹.
12d) cis-(1RS)-1-Formyl-(2RS)-2-[(1E,3E)-(5RS)-5-acetoxy-1,3-tridecadienyl]-cyclohexan
   In Analogie zu Beispiel 1d) erhält man aus 4,2 g des nach Beispiel 12c) hergestellten Acetats 2,9 g des Aldehyds als Rohprodukt.

### Beispiel 13

### (+)-(5RS)-5-Acetoxy-5-[cis-(2RS)-2-((1E,3E)-(5RS)-5-acetoxy-1,3-tridecadienyl)-(1RS)-cyclohex-1-yl]-pentansäure Diastereomeres A

In Analogie zu Beispiel 2 erhält man aus 335 mg des nach Beispiel 12 hergestellten unpolaren diastereomeren Diacetats 230 mg der Titelverbindung als farbloses Öl.
- IR:: 3520 (breit), 2930, 2860, 1725, 1373, 1251, 991, 948 cm⁻¹.

### Beispiel 14

### (+)-(5RS)-5-Hydroxy-5-[cis-(2RS)-2-((1E,3E)-(5RS)-5-hydroxy-1,3-tridecadienyl)-(1RS)-cyclohex-1-yl]-pentansäure Diastereomeres A

In Analogie zu Beispiel 3 erhält man aus 390 mg des nach Beispiel 13 hergestellten Diacetats (Diastereomeres A) 253 mg der Titelverbindung als farbloses Öl.
- IR:: 3600, 3410 (breit), 2930, 2860, 1720, 1375, 993 cm⁻¹.

### Beispiel 15

### (+)-(5RS)-5-Acetoxy-5-[cis-(2RS)-2-((1E,3E)-(5RS)-5-hydroxy-1,3-tridecadienyl)-(1RS)-cyclohex-1-yl]-pentansäure Diastereomeres A

In Analogie zu Beispiel 4 erhält man aus 110 mg des nach Beispiel 13 hergestellten Diacetats (Diastereomeres A) 77 mg der Titelverbindung als farbloses Öl.
- IR:: 3600, 3430, 2929, 2858, 1722, 1375, 1250, 992 cm⁻¹.

### Beispiel 16

### (+)-(5RR)-5-Acetoxy-5-[cis-(2RS)-2-((1E,3E)-(5RS)-5-acetoxy-1,3-tridecadienyl)-(1RS)-cyclohex-1-yl]-pentansäure Diastereomeres B

In Analogie zu Beispiel 2 erhält man aus 270 mg des nach Beispiel 12 hergestellten polaren diastereomeren Diacetats 190 mg der Titelverbindung als farbloses Öl.
- IR:: 3600 (breit), 2930, 2859, 1725, 1375, 1250, 991, 948 cm⁻¹.

### Beispiel 17

### (+)-(5RS)-5-Hydroxy-5-[cis-(2RS)-2-((1E,3E)-(5RS)-5-hydroxy-1,3-tridecadienyl)-(1RS)-cyclohex-1-yl]-pentansäure Diastereomeres B

In Analogie zu Beispiel 3 erhält man 280 mg des nach Beispiel 16 hergestellten Diacetats (Diastereomeres B) 185 mg der Titelverbindung als farbloses Öl.
- IR:: 3610, 3415 (breit), 2930, 2860, 1720, 1375, 993 cm⁻¹.

### Beispiel 18

### (+)-(5RS)-5-Acetoxy-5-[cis-(2RS)-2-((1E,3E)-(5RS)-5-hydroxy-1,3-tridecadienyl)-(1RS)-cyclohex-1-yl]-pentansäure Diastereomeres B

In Analogie zu Beispiel 4 erhält man aus 140 mg des nach Beispiel 16 hergestellten Diacetats (Diastereomeres B) 80 mg der Titelverbindung als farbloses Öl.
- IR:: 3600, 3420, 2930, 2859, 1722, 1376, 1251, 992 cm⁻¹.

### Beispiel 19

### (+)-(5RS)-5-Acetoxy-5-[cis-(6RS)-6-((1E,3E)-(5RS)-5-acetoxy-1,3-tridecadienyl)-(1RS)-3-cyclohexen-1-yl]-pentansäuremethylester Diastereomeres A

Zu einer Lösung von 51 mg der nach Beispiel 2 hergestellten Säure in 5 ml Methylenchlorid tropft man bei 0 °C eine ätherische Diazomethanlösung bis zur bleibenden Gelbfärbung und rührt 15 Minuten bei 0 °C. Anschließend dampft man im Vakuum ein und chromatographiert den Rückstand an Kieselgel. Mit Hexan/Essigester (9+1) erhält man 48 mg der Titelverbindung als farbloses Öl.
- IR (Film):: 2923, 2851, 1739, 1655, 1370, 1240, 990 cm⁻¹.

### Beispiel 20

### (+)-(5RS)-5-Hydroxy-5-[cis-(6RS)-6-((1E,3E)-(5RS)-5-hydroxy-1,3-tridecadienyl)-(1RS)-3-cyclohexen-1-yl]-pentansäuremethylester Diastereomeres A

Zu einer Lösung von 40 mg der nach Beispiel 3 hergestellten Säure in 4 ml Methylenchlorid tropft man bei 0 °C eine ätherische Diazomethanlösung bis zur bleibenden Gelbfärbung und rührt 15 Minuten bei 0 °C. Anschließend dampft man im Vakuum ein und chromatographiert den Rückstand an Kieselgel. Mit Hexan/Essigester (1+9) erhält man 32 mg der Titelverbindung als farbloses Öl.
- IR (Film):: 3610, 2922, 2853, 1737, 1655, 1435, 990 cm⁻¹.

### Beispiel 21

### (+)-(5RS)-5-Acetoxy-5-[cis-(6RS)-6-((1E,3E)-(5RS)-5-hydroxy-1,3-tridecadienyl)-(1RS)-3-cyclohexen-1-yl]-pentansäuremethylester Diastereomeres A

Zu einer Lösung von 38 mg der nach Beispiel 4 hergestellten Säure in 4 ml Methylenchlorid tropft man bei 0 °C eine ätherische Diazomethanlösung bis zur bleibenden Gelbfärbung und rührt 15 Minuten bei 0 °C. Anschließend dampft man im Vakuum ein und chromatographiert den Rückstand an Kieselgel. Mit Hexan/Essigester (1+1) erhält man 30 mg der Titelverbindung als farbloses Öl.
- IR (Film):: 3420, 2923, 2858, 1739, 1655, 1370, 1240, 990 cm⁻¹.

### Beispiel 22

### (+)-(5RS)-5-Acetoxy-5-[cis-(2RS)-2-((1E,3E)-(5RS)-5-acetoxy-1,3-tridecadienyl)-(1RS)-cyclohex-1-yl]-pentansäuremethylester Diastereomeres A

In Analogie zu Beispiel 19 erhält man aus 85 mg der nach Beispiel 13 hergestellten Säure 71 mg der Titelverbindung als farbloses Öl.
- IR (Film):: 2924, 2852, 1739, 1655, 1371, 1240, 991 cm⁻¹.

### Beispiel 23

### (+)-(5RS)-5-Hydroxy-5-[cis-(2RS)-2-((1E,3E)-(5RS)-5-hydroxy-1,3-tridecadienyl)-(1RS)-cyclohex-1-yl]-pentansäuremethylester Diastereomeres A

In Analogie zu Beispiel 20 erhält man aus 73 mg der nach Beispiel 14 hergestellten Säure 65 mg der Titelverbindung als farbloses Öl.
- IR (Film):: 3600, 2922, 2852, 1738, 1655, 1436, 991 cm⁻¹.

### Beispiel 24

### (+)-(5RS)-5-Acetoxy-5-[cis-(2RS)-2-((1E,3E)-(5RS)-5-hydroxy-1,3-tridecadienyl)-(1RS)-cyclohex-1-yl]-pentansäuremethylester Diastereomeres A

In Analogie zu Beispiel 21 erhält man aus 45 mg der nach Beispiel 15 hergestellten Säure 40 mg der Titelverbindung als farbloses Öl.
- IR (Film):: 3430, 2924, 2859, 1739, 1655, 1370, 1240, 990 cm⁻¹.

### Beispiel 25

### (+)-(5RS)-5-Hydroxy-5-[cis-(6RS)-6-[(1E,3E)-(5RS)-5-hydroxy-1,3-tridecadienyl)-(1RS)-3-cyclohexen-1-yl]-pentansäure-1,5-lacton Diastereomeres A

Zu einer Lösung von 40 mg der nach Beispiel 3 hergestellten Säure in 8 ml Toluol fügt man bei 24 °C über einen Zeitraum von 24 Stunden portionsweise 1 g wasserfreies Magnesiumsulfat und rührt weitere 24 Stunden bei 24 °C. Anschließend filtriert man und chromatographiert den Eindampfrückstand an Kieselgel. Mit Toluol/Essigester (7+3) eluiert man 22 mg des Lactons als farbloses Öl.
- IR (CHCl₃):: 3600, 2930, 2860, 1725, 1248, 1045, 992 cm⁻¹.

### Beispiel 26

### (+)-(5RS)-5-Hydroxy-5-[cis-(6RS)-6-[(1E,3E)-(5RS)-5-hydroxy-1,3-tridecadienyl)-(1RS-3-cyclohexen-1-yl]-pentansäure-1,5-lacton Diastereomeres B

In Analogie zu Beispiel 25 erhält man aus 67 mg der nach Beispiel 8 hergestellten Säure 32 mg der Titelverbindung als farbloses Öl.
- IR (CHCl₃):: 3600, 2929, 2860, 1725, 1248, 1045, 992 cm⁻¹.

### Beispiel 27

### (+)-(5RS)-5-Hydroxy-5-[cis-(2RS)-2-((1E,3E)-(5RS)-5-hydroxy-1,3-tridecadienyl)-(1RS)-cyclohex-1-yl]-pentansäure-1,5-lacton Diastereomeres A

In Analogie zu Beispiel 25 erhält man aus 120 mg der nach Beispiel 14 hergestellten Säure 52 mg der Titelverbindung als farbloses Öl.
- IR (CHCl₃):: 3620, 2930, 2860, 1725, 1248, 1046, 993 cm⁻¹.

### Beispiel 28

### (+)-(5RS)-5-Hydroxy-5-[cis-(2RS)-2-((1E,3E)-(5RS)-5-hydroxy-1,3-tridecadienyl)-(1RS)-cyclohex-1-yl]-pentansäure-1,5-lacton Diastereomeres B

In Analogie zu Beispiel 25 erhält man aus 80 mg der nach Beispiel 17 hergestellten Säure 41 mg der Titelverbindung als farbloses Öl.
- IR (CHCl₃):: 3600, 2930, 2860, 1726, 1248, 1046, 993 cm⁻¹.

### Beispiel 29

### (+)-(5RS)-5-Hydroxy-5-[cis-(6RS)-6-((1E,3E)-(5RS)-5-hydroxy-1,3-tridecadienyl)-(1RS)-3-cyclohexen-1-yl]-pentansäure-tris-(hydroxymethyl)-aminomethan-salz

Zu einer Lösung von 38 mg der nach Beispiel 3 hergestellten Carbonsäure in 6 ml Acetonitril fügt man bei 70 °C eine Lösung von 14 mg Tris-(hydroxymethyl)-aminomethan in 0,04 ml Wasser. Man läßt unter Rühren abkühlen, decantiert nach 16 Stunden vom Lösungsmittel und trocknet den Rückstand im Vakuum. Man isoliert 25 mg der Titelverbindung als wachsartige Masse.

### Beispiel 30

### (+)-(5RS)-5-Acetoxy-5-[trans(6RS)-6-((1E,3E)-(5RS)-5-acetoxy-1,3-tridecadienyl)-(1RS)-3-cyclohexen-1-yl]-pentanol unpolares und polares Diastereomeres

Zu 3,65 g Magnesium tropft man bei 25 °C unter Argon eine Lösung aus 16,7 g 4-Chlor-1-(tert.-butyldimethylsilyloxy)-butan in 15 ml Tetrahydrofuran und 0,45 ml Dibrommethan, erwärmt 10 Minuten auf 70 °C, rührt 30 Minuten bei 25 °C und verdünnt mit 45,2 ml Tetrahydrofuran.

Zu 10 ml dieser magnesiumorganischen Lösung tropft man bei -20 °C unter Argon eine Lösung aus 1,95 g trans-(1RS)-1-Formyl-(6RS)-6-[(1E,3E)-(5RS)-5-acetoxy-1,3-tridecadienyl]-3-cyclohexen in 5 ml Tetrahydrofuran und rührt 30 Minuten bei -20 °C. Man versetzt mit 100 ml gesättigter Ammoniumchloridlösung, extrahiert dreimal mit Ether, schüttelt die organische Phase mit Sole, trocknet über Magnesiumsulfat und dampft im Vakuum ein. Den Rückstand chromatographiert man an Kieselgel. Mit Hexan/Essigester (8+2) erhält man 1,85 g des Alkohols als farbloses Öl.
- IR (CHCl₃):: 3600, 2930, 2860, 1725, 1252, 992, 838 cm⁻¹.
Zur Acetylierung fügt man zu einer Lösung aus 1,5 g des vorstehend beschriebenen Alkohols in 30 ml Pyridin 5 ml Essigsäureanhydrid und rührt 16 Stunden bei 24 °C. Anschließend engt man im Vakuum unter Zusatz von Toluol ein und chromatographiert den Rückstand an Kieselgel. Mit Hexan/Essigester (95+5) erhält man 1,64 g des Acetats als farbloses Öl.
- IR:: 2930, 2860, 1728, 1255, 990, 838 cm⁻¹.
Zur Schuztzgruppenabspaltung rührt man 1,2 g des vorstehend hergestellten Acetats in 85 ml Tetrahydrofuran mit 2,0 g Tetrabutylammoniumfluorid 1 Stunde bei 0 °C und 4 Stunden bei 24 °C unter Argon. Anschließend verdünnt man mit Äther, wäscht dreimal mit Wasser, trocknet über Magnesiumsulfat und dampft im Vakuum ein. Den Rückstand chromatographiert man mit Hexan/Essigester (6+4) an Kieselgel. Dabei erhält man zunächst 0,45 g des unpolaren Diastereomeren und dann 0,4 g des polareren Diastereomeren der Titelverbindung als farblose Öle.
- IR (unpolares Diastereomeres):: 3600, 3430, 2930, 2858, 1728, 1608, 1375, 1250, 992 cm⁻¹.
- IR (polares Diastereomeres):: 3620, 3480, 2930, 2860, 1729, 1608, 1375, 1250, 992 cm⁻¹.
Das Ausgangsmaterial für die obige Titelverbindung wird wie folgt hergestellt:
30a) 5-[trans-1-(tert.-butyl-dimethylsilyloxymethyl)-3-cyclohexen-6-yl]-(2E,4E)-pentadiensäureäthylester
   Zu einer Suspension von 10 g Lithiumaluminiumhydrid in 140 ml Tetrahydrofuran tropft man bei Raumtemperatur eine Lösung von 25 g trans-4-cyclohexen-1,2-dicarbonsäurediäthylester in 140 ml Tetrahydrofuran und rührt die Mischung anschließend 3 Stunden bei Rückflußtemperatur. Man kühlt auf 0 °C, tropft langsam eine Mischung aus Tetrahydrofuran/Wasser (1+1) zu, rührt 30 Minuten, versetzt mit 150 ml Chloroform, filtriert und dampft das Filtrat im Vakuum ein. Den Rückstand destilliert man bei 0,2 mm/Hg und 150 °C am Kugelrohr. Dabei erhält man 20 g trans-1,6-Dihydroxymethyl-3-cyclohexen als farbloses Öl.
   Zu einer Lösung von 10 g des vorstehend hergestellten Diols und 9,6 g Imidazol in 93 ml Dimethylformamid fügt man bei 0 °C unter Argon 10,6 g tert.-Butyldimethylsilylchlorid und rührt 16 Stunden bei 25 °C. Man verdünnt mit 0,6 l Äther , schüttelt zweimal mit je 100 ml 10 %iger Schwefelsäure, wäscht mit Sole neutral, trocknet über Magnesiumsulfat und dampft im Vakuum ein. Den Rückstand chromatographiert man an Kieselgel. Mit Hexan/Essigester (9+1) erhält man 7,3 g trans-1-(tert.-Butyl-dimethylsilyloxymethyl)-6-hydroxymethyl-3-cyclohexen als farbloses Öl.
   - IR:: 3600, 3390, 2930, 2858, 835 cm⁻¹.
   Zu einer Lösung von 6,6 g des vorstehend beschriebenen Monosilylathers in 350 ml Methylenchlorid fügt man bei 0 °C 45 g Collins-Reagenz (Chromsäure-Pyridin-Komplex) und rührt 40 Minuten bei 0 °C. Anschließend verdünnt man mit einer Mischung aus Hexan/Äther (3+2), fügt Celite hinzu, filtriert und dampft im Vakuum ein. Den Rückstand chromatographiert man mit Hexan/Essigester (9+1) an Kieselgel. Dabei erhält man 5,5 g trans-1-(tert.-Butyl-dimethylsilyloxymethyl)-6-formyl-3-cyclohexen als farbloses Öl.
   - IR:: 2930, 2860, 2730, 1716, 838 cm⁻¹.
   Zur Wittig-Horner-Olefinierung fügt man bei 24 °C 4,6 g Phosphonocrotonsäuretriäthylester und 2,3 g Diazabicycloundecen (DBU) zu einer gerührten Suspension von 0,78 g Lithiumchlorid in 153 ml Acetonitril und rührt 10 Minuten. Anschließend tropft man eine Lösung aus 3,9 g des vorstehend beschriebenen Aldehyds in 31 ml Acetonitril zu, rührt 3 Stunden bei 24 °C und verdünnt dann mit Äther. Man schüttelt nacheinander mit Wasser, 10 %iger Citronensäurelösung und Wasser, trocknet über Magnesiumsulfat und dampft im Vakuum ein. Den Rückstand chromatographiert man mit Hexan/Essigester (95+5) an Kieselgel. Dabei erhält man 4,1 g der Titelverbindung als farbloses Öl.
   - IR:: 2930, 2858, 1710, 1638, 1618, 1256, 1003, 940, 838 cm⁻¹.
30b) 5-[trans-1-(tert.-Butyl-dimethylsilyloxymethyl)-3-cyclohexen-6-yl]-(2E,4E)-pentadien-1-al
   Zu einer Lösung von 3,5 g des nach Beispiel 30a) hergestellten Esters in 94 ml Toluol tropft man bei -70 °C unter Argon 16,6 ml einer 1,2 molaren Lösung von Diisobutylaluminiumhydrid in Toluol und rührt 40 Minuten bei -70 °C. Anschließend tropft man 2 ml Isopropanol und dann 8 ml Wasser zu, rührt 2 Stunden bei 22 °C, filtriert, wäscht mit Methylenchlorid und dampft im Vakuum ein. Den Rückstand reinigt man durch Chromatographie an Kieselgel. Mit Hexan/Essigester (8+2) erhält man 3,04 g des Alkohols als farbloses Öl.
   - IR :: 3620, 3460, 838 cm⁻¹.

   Eine Lösung von 3,02 g des vorstehend hergestellten Alkohols in 110 ml Toluol versetzt man mit 8,5 g Braunstein und rührt 4,5 Stunden bei 24 °C. Anschließend wird filtriert, eingedampft und an Kieselgel chromatographiert. Mit Hexan/Essigester (85+15) eluiert man 2,9 g der Titelverbindung als farbloses Öl.
   - IR:: 2929, 2860, 1680, 1638, 992, 950, 838 cm⁻¹.
30c) (5RS)-5-Acetoxy-1-[trans-1-(tert.-Butyl-dimethylsilyloxymethyl)-3-cyclohexen-6-yl]-(1E,3E)-tridecadien
   Zu 1,2 g Magnesium in 5 ml Äther tropft man unter Erwärmen eine Lösung aus 7,95 ml Octylbromid in 12 ml Äther und rührt 30 Minuten bei 25 °C.
   Zu 5,85 ml (= 10,77 mMol) dieser Grignard-Lösung tropft man bei -20 °C unter Argon eine Lösung aus 2,85 g des nach Beispiel 30b) hergestellten Aldehyds in 47 ml Äther und rührt 3 Stunden bei -20 °C. Man versetzt mit gesättigter Ammoniumchloridlösung, extrahiert dreimal mit Äther, schüttelt die organische Phase mit Sole, trocknet über Magnesiumsulfat und dampft im Vakuum ein. Den Rückstand chromatographiert man an Kieselgel. Mit Hexan/Essigester (7+3) eluiert man 2,92 g des Alkohols als farbloses Öl.
   - IR:: 3620, 3460, 2929, 2858, 992, 838.

   Zur Acetylierung fügt man zu einer Lösung von 3,0 g des vorstehend hergestellten Alkohols in 30 ml Pyridin 5 ml Essigsäureanhydrid und rührt 16 Stunden bei 24 °C. Anschließend engt man im Vakuum unter Zusatz von Toluol ein und chromatographiert den Rückstand an Kieselgel. Mit Hexan/Essigester (8+2) erhält man 3,25 g der Titelverbindung als Öl.
   - IR:: 2930, 2858, 1730, 1254, 990, 838 cm⁻¹.
30d) trans-(1RS)-1-Formyl-(6RS)-6-[(1E,3E)-(5RS)-5-acetoxy-1,3-tridecadienyl]-3-cyclohexen
   Zu einer Lösung von 3,2 g des nach Beispiel 30c) hergestellten Acetats in 320 ml Tetrahydrofuran fügt man bei 0 °C 6,55 g Tetrabutylammoniumfluorid, rührt 1 Stunde bei 0 °C und 3 Stunden bei 24 °C. Anschließend verdünnt man mit 1,2 l Äther und wäscht dreimal mit Sole. Man trocknet über Magnesiumsulfat, dampft im Vakuum ein und chromatographiert den Rückstand an Kieselgel. Mit Hexan/Essigester (6+4) eluiert man 2,34 g des Alkohols als farbloses Öl.
   - IR:: 3620, 3458, 2930, 2860, 1722, 1250, 991 cm⁻¹.

   Zu einer Lösung von 2,46 g des vorstehend hergestellten Alkohols in 70 ml Methylenchlorid fügt man bei 0 °C 18 g Collins-Reagenz (Chromsäure-Pyridin-Komplex) und rührt 15 Minuten bei 0 °C. Anschließend verdünnt man mit einer Mischung aus Hexan/Äther (3+2), fügt Celite hinzu, filtriert und dampft im Vakuum ein. Der so erhaltene Aldehyd wird ohne weitere Reinigung verwendet.

### Beispiel 31

### (+)-(5RS)-5-Acetoxy-5-[trans-(6RS)-6-((1E,3E)-(5RS)-5-acetoxy-1,3-tridecadienyl)-(1RS)-3-cyclohexen-1-yl]-pentansäure Diastereomeres A

Zu einer Lösung von 360 mg des in Beispiel 30 beschriebenen unpolaren diastereomeren Diacetats in 28 ml Methylenchlorid fügt man bei 0 °C 1,98 g Collins-Reagenz und rührt 15 Minuten bei 0 °C. Anschließend verdünnt man mit einer Mischung aus Hexan/Äther (3+2), fügt Celite hinzu, filtriert, wäscht mit Hexan/Äther (3+2) und dampft im Vakuum ein.

Zu einer Lösung von 380 mg des vorstehend hergestellten Aldehyds in 12,5 ml Aceton tropft man unter Rühren bei -20 °C 0,7 ml Jones-Reagenz (J. Chem. Soc. 1953, 2555) und rührt 12 Minuten bei -20 °C unter Argon. Anschließend fügt man 3 ml Isopropanol zu, rührt 10 Minuten, verdünnt mit 40 ml Ether, filtriert, wäscht mit Ether, schüttelt die Etherphase zweimal mit Sole, trocknet über Magnesiumsulfat und dampft im Vakuum ein. Den Rückstand chromatographiert man an Kieselgel. Mit Hexan/Essigester (6+4) erhält man 280 mg der Titelverbindung als farbloses Öl.
- IR:: 3520 (breit), 2928, 2859, 1725, 1372, 1250, 991, 948 cm⁻¹.

### Beispiel 32

### (+)-(RS)-5-Hydroxy-5-[trans-(6RS)-6-((1E,3E)-(5RS)-5-hydroxy-1,3-tridecadienyl)-(1RS)-3-cyclohexen-1-yl]-pentansäure Diastereomeres A

68 mg des in Beispiel 31 beschriebenen unpolaren diasteremoren Diacetats (Diastereomeres A) rührt man 48 Stunden bei 24 °C mit 2,5 ml einer Lösung von Kaliumhydroxid in Wasser und Äthanol (Herstellung: man löst 5 g Kaliumhydroxid in 67,5 ml Wasser und 182,5 ml Äthanol). Anschließend säuert man mit 10 %iger Zitronensäurelösung auf pH4 an, extrahiert viermal mit je 15 ml Methylenchlorid, schüttelt die organische Phase mit Sole, trocknet über Magnesiumsulfat und dampft im Vakuum ein. Den Rückstand chromatographiert man mit Essigester/Hexan (8+2) an Kieselgel. Dabei erhält man 41 mg der Titelverbindung als farbloses Öl.
- IR:: 3620, 3415 (breit), 2929, 2859, 1720, 1375, 992 cm⁻¹.

### Beispiel 33

### (+)-(5RS)-5-Acetoxy-5-[trans-(6RS)-6-((1E,3E)-(5RS)-5-hydroxy-1,3-tridecadienyl)-(1RS)-3-cyclohexen-1-yl]-pentansäure Diastereomeres A

Zu einer Lösung von 72 mg des nach Beispiel 31 hergestellten unpolaren diastereomeren Diacetats (Diastereomeres A) in 1,5 ml Methanol fügt man bei 24 °C 1,5 ml einer 0,5 normalen Natronlauge und rührt 7 Stunden bei 24 °C unter Argon. Anschließend verdünnt man mit 2 ml Wasser und säuert bei Eisbadtemperatur mit 10 %iger Zitronensäurelösung auf pH4 an. Man extrahiert viermal mit je 30 ml Essigester, schüttelt die organische Phase mit Sole, trocknet über Magnesiumsulfat und dampft im Vakuum ein. Den Rückstand chromatographiert man an Kieselgel. Mit Äther/Hexan (1+1) erhält man 51 mg der Titelverbindung als farbloses Öl.
- IR:: 3615, 3450, 2930, 2859, 1721, 1375, 1250, 992 cm⁻¹.

### Beispiel 34

### (+)-(5RS)-5-Hydroxy-5-[trans-(6RS)-6-((1E,3E)-(5RS)-5-hydroxy-1,3-tridecadienyl)-(1RS)-3-cyclohexen-1-yl]-pentanol Diastereomeres A

98 mg des in Beispiel 30 beschriebenen unpolaren diastereomeren Diacetats rührt man 60 Stunden bei 24 °C mit 2,9 ml einer Lösung von Kaliumhydroxid in Wasser und Äthanol (Herstellung: man löst 5 g Kaliumhydroxid in 67,5 ml Wasser und 182,5 ml Äthanol). Anschließend säuert man mit 10 %iger Zitronensäurelösung auf pH6 an, extrahiert viermal mit je 20 ml Methylenchlorid, schüttelt die organische Phase mit Sole, trocknet über Magnesiumsulfat und dampft im Vakuum ein. Den Rückstand chromatographiert man mit Essigester an Kieselgel. Dabei erhält man 58 mg der Titelverbindung als farbloses Öl.
- IR:: 3600, 3380 (breit), 2930, 2860, 992 cm⁻¹.

### Beispiel 35

### (+)-(5RS)-5-Acetoxy-5-[trans-(6RS)-6-((1E,3E)-(5RS)-5-hydroxy-1,3-tridecadienyl)-(1RS)-3-cyclohexen-1-yl]-pentanol Diasteremoeres A

60 mg des in Beispiel 30 beschriebenen unpolaren diasteremoeren Diacetats in 1,4 ml Methanol rührt man 4 Stunden bei 24 °C mit 1,4 ml einer 0,5 normalen Natronlauge unter Argon. Anschließend verdünnt man mit 5 ml Wasser, neutralisiert mit 10 %iger Zitronensäurelösung, extrahiert viermal mit je 20 ml Methylenchlorid, schüttelt die organische Phase mit Sole, trocknet über Magnesiumsulfat und dampft im Vakuum ein. Den Rückstand chromatographiert man mit Essig/Hexan (1+1) an Kieselgel. Dabei erhält man 21 mg der Titelverbindung als farbloses Öl.
- IR:: 3600, 3450 (breit), 2929, 2860, 1730, 1375, 1252, 993 cm⁻¹.

### Beispiel 36

### (+)-(5RS)-5-Acetoxy-5-[trans-(6RS)-6-((1E,3E)-(5RS)-5-Acetoxy-1,3-tridecadienyl)-(1RS)-3-cyclohexen-1-yl]-pentansäure Diastereomeres B

In Analogie zu Beispiel 31 erhält man aus 405 mg des nach Beispiel 30 hergestellten polaren diastereomeren Diacetats 300 mg der Titelverbindung als farbloses Öl.
- IR:: 3515 (breit), 2929, 2860, 1725, 1372, 1250, 991, 948 cm⁻¹.

### Beispiel 37

### (+)-(5RS)-5-Hydroxy-5-[trans-(6RS)-6-((1E,3E)-(5RS)-5-hydroxy-1,3-tridecadienyl)-(1RS)-3-cyclohexen-1-yl]-pentansäure Diastereomeres B

In Analogie zu Beispiel 32 erhält man aus 220 mg des nach Beispiel 36 hergestellten Diacetats (Diastereomeres B) 160 mg der Titelverbindung als farbloses Öl.
- IR:: 3600, 3420 (breit), 2930, 2860, 1721, 1375, 992 cm⁻¹.

### Beispiel 38

### (+)-(5RS)-5-Acetoxy-5-[trans-(6RS)-6-((1E,3E)-(5RS)-5-hydroxy-1,3-tridecadienyl)-(1RS)-3-cyclohexen-1-yl]-pentansäure Diastereomeres B

In Analogie zu Beispiel 33 erhält man aus 120 mg des nach Beispiel 36 hergestellten Diacetats (Diastereomeres B) 60 mg der Titelverbindung als farbloses Öl.
- IR:: 3620, 3400 (breit), 2930, 2860, 1722, 1375, 1250, 992 cm⁻¹.

### Beispiel 39

### (+)-(5RS)-5-Hydroxy-5-[trans-(6RS)-6-((1E,3E)-(5RS)-5-hydroxy-1,3-tridecadienyl)-(1RS)-3-cyclohexen-1-yl]-pentanol Diastereomeres B

In Analogie zu Beispiel 34 erhält man aus 140 mg des in Beispiel 30 hergestellten polaren diastereomeren Diacetats 72 mg der Titelverbindung als farbloses Öl.
- IR:: 3600, 3420 (breit), 2930, 2860, 992 cm⁻¹.

### Beispiel 40

### (+)-(5RS)-5-Acetoxy-5-[trans-(6RS)-6-((1E,3E)-(5RS)-5-hydroxy-1,3-tridecadienyl)-(1RS)-3-cyclohexen-1-yl]-pentanol Diastereomeres B

In Analogie zu Beispiel 35 erhält man aus 50 mg des in Beispiel 30 hergestellten polaren diastereomeren Diacetats 30 mg der Titelverbindung als farbloses Öl.
- IR:: 3620, 3430 (breit), 2930, 2860, 1729, 1376, 1250, 993 cm⁻¹.

### Beispiel 41

### (+)-(5RS)-5-Acetoxy-5-[trans-(2RS)-2-((1E,3E)-(5RS)-5-acetoxy-1,3-tridecadienyl)-(1RS)-cyclohex-1-yl]-pentanol unpolares und polares Diastereomeres

In Analogie zu Beispiel 30 erhält man aus 2 g trans-(1RS)-1-Formyl-(2RS)-2-[(1E,3E)-(5RS)-5-acetoxy-1,3-tridecadienyl]-cyclohexan 0,5 g des unpolareren Diastereomeren und 0,6 g des polareren Diastereomeren der Titelverbindung als farblose Öle.
- IR (unpolares Diastereomeres):: 3620, 3420, 2929, 2859, 1729, 1607, 1375, 1250, 992 cm⁻¹.
- IR (polares Diastereomeres):: 3600, 3430, 2929, 2860, 1729, 1607, 1375, 1250, 992 cm⁻¹.

Das Ausgangsmaterial für die obige Titelverbindung wird wie folgt hergestellt:
41a) 5-[trans-1-(tert.-Butyl-dimethylsilyloxymethyl)-cyclohex-2-yl]-(2E,4E)-pentadiensäureäthylester
   In Analogie zu Beispiel 30a) erhält man aus trans-1,2-Dihydroxymethyl-cyclohexan die Titelverbindung als farbloses Öl. IR: 2930, 2859, 1710, 1638, 1618, 1255, 1004, 940, 838 cm⁻¹.
41b) 5-[trans-1-(tert.-Butyl-dimethylsilyloxymethyl)-cyclohex-2-yl]-(2E,4E)-pentadien-1-al
   In Analogie zu Beispiel 30b) erhält man aus 33 g des nach Beispiel 41a) hergestellten Esters 28 g der Titelverbindung als farbloses Öl. IR: 2930, 2859, 1680, 1640, 993, 950, 840 cm⁻¹.
41c) (5RS)-5-Acetoxy-1-[trans-1-(tert.-Butyldimethyl-silyloxymethyl)-cyclohex-1-yl]-(1E,3E)-tridecadien
   In Analogie zu Beispiel 30c) erhält man aus 8,3 g des nach Beispiel 41b) hergestellten Aldehyds 7,5 g der Titelverbindung als farbloses Öl. IR: 2930, 2860, 1731, 1255, 990, 838 cm⁻¹.
41d) trans-(1RS)-1-Formyl-(2RS)-2-[(1E,3E)-(5RS)-5-acetoxy-1,3-tridecadienyl]-cyclohexan
   In Analogie zu Beispiel 30d) erhält man aus 4,3 g des nach Beispiel 30c) hergestellten Acetats 3,0 g des Aldehyds als Rohprodukt.

### Beispiel 42

### (+)-(5RS)-5-Acetoxy-5-[trans-(2RS)-2-((1E,3E)-(5RS)-5-acetoxy-1,3-tridecadienyl)-(1RS)-cyclohex-1-yl]-pentansäure Diastereomeres A

In Analogie zu Beispiel 31 erhält man aus 500 mg des nach Beispiel 41 hergestellten unpolaren diastereomeren Diacetats 390 mg der Titelverbindung als - farbloses Öl.
- IR:: 3500 (breit), 2930, 2860, 1725, 1373, 1251, 991, 948 cm⁻¹.

### Beispiel 43

### (+)-(5RS)-5-Hydroxy-5-[trans-(2RS)-2-((1E,3E)-(5RS)-5-hydroxy-1,3-tridecadienyl)-(1RS)-cyclohex-1-yl]-pentansäure Diastereomeres A

In Analogie zu Beispiel 32 erhält man aus 300 mg des nach Beispiel 42 hergestellten Diacetats (Diastereomeres A) 203 mg der Titelverbindung als farbloses Öl.
- IR:: 3620, 3410 (breit), 2930, 2859, 1720, 1375, 993 cm⁻¹.

### Beispiel 44

### (+)-(5RS)-5-Acetoxy-5-[trans-(2RS)-2-((1E,3E)-(5RS)-5-hydroxy-1,3-tridecadienyl)-(1RS)-cyclohex-1-yl]-pentansäure Diastereomeres A

In Analogie zu Beispiel 33 erhält man aus 120 mg des nach Beispiel 42 hergestellten Diacetats (Diastereomeres A) 70 mg der Titelverbindung als farbloses Öl.
- IR:: 3620, 3430, 2929, 2859, 1722, 1375, 1250, 992 cm⁻¹.

### Beispiel 45

### (+)-(5RR)-5-Acetoxy-5-[trans-(2RS)-2-((1E,3E)-(5RS)-5-acetoxy-1,3-tridecadienyl)-(1RS)-cyclohex-1-yl]-pentansäure Diastereomeres B

In Analogie zu Beispiel 31 erhält man aus 290 mg des nach Beispiel 41 hergestellten polaren diastereomeren Diacetats 180 mg der Titelverbindung als farbloses Öl.
- IR:: 3620 (breit), 2930, 2860, 1725, 1375, 1250, 991, 948 cm⁻¹.

### Beispiel 46

### (+)-(5RS)-5-Hydroxy-5-[trans-(2RS)-2-((1E,3E)-(5RS)-5-hydroxy-1,3-tridecadienyl)-(1RS)-cyclohex-1-yl]-pentansäure Diastereomeres B

In Analogie zu Beispiel 32 erhält man 310 mg des nach Beispiel 45 hergestellten Diacetats (Diastereomeres B) 205 mg der Titelverbindung als farbloses Öl.
- IR:: 3620, 3415 (breit), 2930, 2860, 1720, 1375, 993 cm⁻¹.

### Beispiel 47

### (+)-(5RS)-5-Acetoxy-5-[trans-(2RS)-2-((1E,3E)-(5RS)-5-hydroxy-1,3-tridecadienyl)-(1RS)-cyclohex-1-yl]-pentansäure Diastereomeres B

In Analogie zu Beispiel 33 erhält man aus 150 mg des nach Beispiel 45 hergestellten Diacetats (Diastereomeres B 70 mg der Titelverbindung als farbloses Öl.
- IR:: 3600, 3410, 2930, 2860, 1722, 1376, 1251, 992 cm⁻¹.

### Beispiel 48

### (+)-(5RS)-5-Acetoxy-5-[trans-(6RS)-6-((1E,3E)-(5RS)-5-acetoxy-1,3-tridecadienyl)-(1RS)-3-cyclohexen-1-yl]-pentansäuremethylester Diastereomeres A

Zu einer Lösung von 150 mg der nach Beispiel 31 hergestellten Säure in 15 ml Methylenchlorid tropft man bei 0 °C eine ätherische Diazomethanlösung bis zur bleibenden Gelbfärbung und rührt 15 Minuten bei 0 °C. Anschließend dampft man im Vakuum ein und chromatographiert den Rückstand an Kieselgel. Mit Hexan/Essigester (9+1) erhält man 140 mg der Titelverbindung als farbloses Öl.
- IR (Film):: 2923, 2851, 1739, 1655, 1370, 1240, 990 cm⁻¹.

### Beispiel 49

### (+)-(5RS)-5-Hydroxy-5-[trans-(6RS)-6-((1E,3E)-(5RS)-5-hydroxy-1,3-tridecadienyl)-(1RS)-3-cyclohexen-1-yl]-pentansäuremethyester Diastereomeres A

Zu einer Lösung von 80 mg der nach Beispiel 32 hergestellten Säure in 8 ml Methylenchlorid tropft man bei 0 °C eine ätherische Diazomethanlösung bis zur bleibenden Gelbfärbung und rührt 15 Minuten bei 0 °C. Anschließend dampft man im Vakuum ein und chromatographiert den Rückstand an Kieselgel. Mit Hexan/Essigester (1+9) erhält man 70 mg der Titelverbindung als farbloses Öl.
- IR (Film):: 3620, 2922, 2853, 1738, 1655, 1435, 990 cm⁻¹.

### Beispiel 50

### (+)-(5RS)-5-Acetoxy-5-[trans-(6RS)-6-((1E,3E)-(5RS)-5-hydroxy-1,3-tridecadienyl)-(1RS)-3-cyclohexen-1-yl]-pentansäuremethylester Diastereomeres A

Zu einer Lösung von 48 mg der nach Beispiel 33 hergestellten Säure in 5 ml Methylenchlorid tropft man bei 0 °C eine ätherische Diazomethanlösung bis zur bleibenden Gelbfärbung und rührt 15 Minuten bei 0 °C. Anschließend dampft man im Vakuum ein und chromatographiert den Rückstand an Kieselgel. Mit Hexan/Essigester (1+1) erhält man 38 mg der Titelverbindung als farbloses Öl.
- IR (Film):: 3400, 2923, 2858, 1740, 1655, 1370, 1240, 990 cm⁻¹.

### Beispiel 51

### (+)-(5RS)-5-Acetoxy-5-[trans-(2RS)-2-((1E,3E)-(5RS)-5-acetoxy-1,3-tridecadienyl)-(1RS)-cyclohex-1-yl]-pentansäuremethylester Diastereomeres A

In Analogie zu Beispiel 48 erhält man aus 67 mg der nach Beispiel 42 hergestellten Säure 51 mg der Titelverbindung als farbloses Öl.
- IR (Film):: 2924, 2852, 1740, 1655, 1371, 1240, 991 cm⁻¹.

### Beispiel 52

### (+)-(5RS)-5-Hydroxy-5-[trans-(2RS)-2-((1E,3E)-(5RS)-5-hydroxy-1,3-tridecadienyl)-(1RS)-cyclohex-1-yl]-pentansäuremethylester Diastereomeres A

In Analogie zu Beispiel 49 erhält man aus 29 mg der nach Beispiel 43 hergestellten Säure 22 mg der Titelverbindung als farbloses Öl.
- IR (Film):: 3600, 2922, 2852, 1738, 1655, 1436, 991 cm⁻¹.

### Beispiel 53

### (+)-(5RS)-5-Acetoxy-5-[trans-(2RS)-2-((1E,3E)-(5RS)-5-hydroxy-1,3-tridecadienyl)-(1RS)-cyclohex-1-yl]-pentansäuremethylester Diastereomeres A

In Analogie zu Beispiel 50 erhält man aus 65 mg der nach Beispiel 44 hergestellten Säure 57 mg der Titelverbindung als farbloses Öl.
- IR (Film):: 3420, 2924, 2859, 1740, 1655, 1370, 1240, 990 cm⁻¹.

### Beispiel 54

### (+)-(5RS)-5-Hydroxy-5-[trans-(6RS)-6-[(1E,3E)-(5RS)-5-hydroxy-1,3-tridecadienyl)-(1RS)-3-cyclohexen-1-yl]-pentansäure-1,5-lacton Diastereomeres A

Zu einer Lösung von 50 mg der nach Beispiel 32 hergestellten Säure in 10 ml Toluol fügt man bei 24 °C über einen Zeitraum von 24 Stunden portionsweise 1 g wasserfreies Magnesiumsulfat und rührt weitere 24 Stunden bei 24 °C. Anschließend filtriert man und chromatographiert den Eindampfrückstand an Kieselgel. Mit Toluol/Essigester (7+3) eluiert man 28 mg des Lactons als farbloses Öl.
- IR (CHCl₃):: 3620, 2930, 2860, 1726, 1248, 1045, 992 cm⁻¹.

### Beispiel 55

### (+)-(5RS)-5-Hydroxy-5-[trans-(6RS)-6-[(1E,3E)-(5RS)-5-hydroxy-1,3-tridecadienyl)-(1RS)-3-cyclohexen-1-yl]-pentansäure-1,5-lacton Diastereomeres B

In Analogie zu Beispiel 54 erhält man aus 60 mg der nach Beispiel 37 hergestellten Säure 30 mg der Titelverbindung als farbloses Öl.
- IR (CHCl₃):: 3620, 2930, 2860, 1725, 1248, 1045, 992 cm⁻¹.

### Beispiel 56

### (+)-(5RS)-5-Hydroxy-5-[trans-(2RS)-2-((1E,3E)-(5RS)-5-hydroxy-1,3-trideca-dienyl)-(1RS)-cyclohex-1-yl]-pentansäure-1,5-lacton Diastereomeres A

In Analogie zu Beispiel 54 erhält man aus 100 mg der nach Beispiel 43 hergestellten Säure 40 mg der Titelverbindung als farbloses Öl.
- IR (CHCl₃):: 3600, 2930, 2860, 1726 1248, 1046, 993 cm⁻¹.

### Beispiel 57

### (+)-(5RS)-5-Hydroxy-5-[trans-(2RS)-2-((1E,3E)-(5RS)-5-hydroxy-1,3-tridecadienyl)-(1RS-cyclohex-1-yl]-pentansäure-1,5-lacton Diastereomeres B

In Analogie zu Beispiel 54 erhält man aus 40 mg der nach Beispiel 46 hergestellten Säure 23 mg der Titelverbindung als farbloses Öl.
- IR (CHCl₃):: 3620, 2930, 2860, 1725, 1248, 1046, 993 cm⁻¹.

### Beispiel 58

### (+)-(5RS)-5-Hydroxy-5-[trans-(6RS)-6-((1E,3E)-(5RS)-5-hydroxy-1,3-tridecadienyl)-(1RS)-3-cyclohexen-1-yl]-pentansäure-tris-(hydroxymethyl)-aminomethansalz

Zu einer Lösung von 80 mg der nach Beispiel 23 hergestellten Carbonsäure in 13 ml Acetonitril fügt man bei 70 °C eine Lösung von 29 mg Tris-(hydroxymethyl)aminomethan in 0,04 ml Wasser. Man läßt unter Rühren abkühlen, decantiert nach 16 Stunden vom Lösungsmittel und trocknet den Rückstand im Vakuum. Man isoliert 25 mg der Titelverbindung als wachsartige Masse.

### BEISPIEL 59

### (±)-(5RS)-5-Acetoxy-5-[cis-(2RS)-2-((1E,3E)-(5RS)-5-hydroxy-1,3-trideca-1,3-dien-7-inyl)-(1RS)-cyclohex-1-yl]-pentansäure unpolares und polares Diastereomeres

Zu 480 mg Magnesium tropft man bei 25°C unter Argon eine Lösung von 2,22 g 4-Chlor-1-(tert.-butyldimethylsilyloxy)-butan in 2 ml Tetrahydrofuran und 0,06 ml Dibromethan, erwarmt 10 Minuten auf 60°C, ruhrt 30 Minuten bei 25°C und verdünnt mit 6,2 ml Tetrahydrofuran.
Zu 922 mg cis-(1RS)-1-Formyl-(2RS)-[(1E,3E)-(5RS)-5-tert.-butyldiphenylsilyloxy-trideca-1,3-dien-7-inyl]-cyclohexan gelöst in 5 ml Tetrahydrofuran gibt man bei -70°C unter Argon 4 ml der oben hergestellten Grignard-Lösung und rührt 1 Stunde bei -70°C. Die Reaktionsmischung gibt man auf gesättigte Ammoniumchlorid-Lösung, extrahiert mehrmals mit Ether, wäscht die organische Phase mit Sole, trocknet über Natriumsulfat und dampft im Vakuum ein. Den so erhaltenen Rückstand löst man in 4 ml Pyridin, gibt unter Argon 1 ml Essigsäureanhydrid zu und rührt 24 Stunden bei 24°C. Anschließend dampft man im Vakuum unter Zusatz von Toluol ein. Den Rückstand chromatographiert man an Kieselgel mit Hexan/0-50% Methylenchlorid. Man erhält 487 mg (±)-(5RS)-5-Acetoxy5-[cis-(2RS)-2-(-(1E,3E)-(5RS)-5-tert.-butyldiphenylsilyloxy-trideca-1,3-dien-7-inyl)-(1RS)-cyclohex-1-yl]-pentan-1-ol-tert.-butyldimethylsilylether als farbloses Öl.
- IR:: 2935, 2860, 1730, 1255, 1122, 992, 843, 705 cm⁻¹
Zur selektiven Schutzgruppenspaltung werden 400 mg des vorstehend hergestellten Acetats in 5 ml Ethanol mit 400 mg Pyridinium-p-toluol-sulfonat 1 Stunde bei 60°C unter Argon gerührt. Nach dem Abkühlen auf 24°C verdünnt man mit Ether, wäscht zweimal mit Wasser, trocknet über Natriumsulfat und dampft im Vakuum ein. Den Rückstand chromatographiert man an Kieselgel mit Hexan/ 0-40% Essigester. Man erhalt so 227 mg (±)-(5RS)-5-Acetoxy-5-[cis-(2RS)-2-((1E,3E)-(5RS)-5-tert.-butyldiphenylsilyloxy-trideca-1,3-dien-7-inyl)-(1RS)-cyclohex-1-yl]-pentan-1-ol als farbloses Öl.
- IR:: 3680, 3440, 2935, 2860, 1730, 1255, 1122, 990, 705 cm⁻¹
220 mg des vorstehend hergestellten Alkohols in 22 ml Methylenchlorid versetzt man bei 0°C unter Argon mit 1,76 g Collins-Regenz (Chromsaure-Pyridin-Komplex) und rührt 30 Minuten bei 0°C. Man verdünnt mit Hexan/Ether (1+1), filtriert über Celite ab, wäscht mit Herxan/Ether (1+1) nach und dampft im Vakuum ein.

Zu einer Lösung von 237 mg des vorstehend hergestellten Aldehyds in 10 ml Aceton tropft man unter Rühren bei -30°C 1,0 ml Jones-Reagenz (J. Chem. Soc. 1953, 2555) und rührt 30 Minuten bei -20°C unter Argon. Anschließend fügt man 0,3 ml Isopropanol zu, rührt 10 Minuten bei -20°C, verdünnt mit Ether, wäscht mit Sole/Wasser (1+1) neutral, trocknet über Natriumsulfat und dampft im Vakuum ein. Den Rückstand chromatographiert man an Kieselgel mit Hexan/Essigester (7+3). Man erhält so 171 mg (±)-(5RS)-5-Acetoxy-5-[cis-(2RS)-2-((1E,3E)-(5RS)-5-tert.-butyldiphenylsilyloxy-trideca-1,3-dien-7-inyl)-(1RS)-cyclohex-1-yl]-pentansäure als farbloses Öl.
- IR:: 3520, 3200, 2935, 2860, 1725, 1255, 1120, 990, 703 cm⁻¹
20 mg der vorstehend hergestellten Saure gelost in 1 ml Tetrahydrofuran versetzt man mit 100 mg Tetrabutylammoniumfluorid und rührt über 16 Stunden bei 24°C unter Argon. Anschließend verdünnt man mit Ether, wascht mit Sole, trocknet über Natriumsulfat und dampft im Vakuum ein. Den Rückstand chromatographiert man an Kieselgel mit Hexan/30-60% Essigester. Dabei erhält man zunächst 4,7 mg des unpolaren Diastereomeren und dann 5,5 mg des polaren Distereomeren der Titelverbindung als farblose Öle.
- IR (unpolares Diastereomeres):: 3590, 3410, 2930, 2860, 1725, 1250, 1120, 991 cm⁻¹
- IR (polares Diastereomeres):: 3600, 3400, 2928, 2859, 1730, 1250, 1122, 990 cm⁻¹
Das Ausgangsmaterial für die obige Titelverbindung wird wie folgt hergestellt:

### a) 1-[cis-(2RS)-2-(tert.-Butyldimethylsilyloxymethyl)-(1RS)-cyclohex-1-yl]-(1E, 3E)-(5RS)-octa-1,3-dien-7-in-5-ol

Zu 2,05 g Magnesium in 25 ml Ether werden einige Kristalle Jod und 12,5 mg Quecksilber(II)chlorid gegeben. Von insgesamt 7,5 ml Propargylbromid tropft man solange das pure Bromid bei 24°C zu bis die Grignard-Reaktion angesprungen ist. Dann wird das restliche Bromid in 25 ml Ether gelöst bei 0°C langsam Zugetropft und nach vollständiger Zugabe noch 1 Stunde bei 24°C nachgerührt.

Zu einer Losung von 8,1 g 5-[cis-1-(tert.-Butyldimethylsilyloxymethyl)-cyclohex-2-yl]-(2E,4E)-pentadien-1-al (hergestellt nach Beispiel 12b) in 125 ml Ether gibt man 63 mg Quecksilber(II)chlorid und tropft langsam bei -70°C unter Argon 40 ml der oben hergestellten Grignard-Lösung zu. Anschließend laßt man auf 0°C erwärmen und rührt bei dieser Temperatur weitere 30 Minuten. Die Reaktionsmischung wird dann auf gesättigte Ammoniumchloridlösung gegeben und mehrmals mit Ether extrahiert. Die organische Phase wird mit Sole gewaschen, über Natriumsulfat getrocknet und im Vakuum eingeengt. Den Rückstand chromatographiert man an Kieselgel mit Hexan/0-40% Essigester. Man erhält so 7,48 g der Titelverbindung als farbloses Öl.
- IR:: 3390, 3315, 2930, 2860, 2120, 990, 838 cm-¹

### b) 1-[cis-(2RS)-2-(tert.-Butyldimethylsilyloxymethyl)-(1RS)-cyclohex-1-yl)-(1E, 3E)-(5RS)-octa-1,3-dien-7-in-5-ol-tert.-butyldiphenylsilylether

7,48 g des vorstehend hergestellten Alkohols gelöst in 130 ml Dimethylformamid werden mit 4,64 g Imidazol versetzt. Zu dieser Lösung gibt man bei 0°C unter Argon 7,97 g tert.-Butyl-diphenylsilylchlorid und rührt 16 Stunden bei 24°C. Anschließend gibt man das Reaktionsgemisch auf Wasser, extrahiert mehrmals mit Hexan/Ether (1+1), trocknet über Natriumsulfat und engt im Vakuum ein. Den Rückstand chromatographiert man an Kieselgel mit Hexan/0-30% Ether. Man erhält so 11,7 g der Titelverbindung als farbloses Öl.
- IR:: 3320, 2925, 2862, 2125, 1120, 992, 840, 705 cm⁻¹

### c) 1-[cis-(2RS)-2-(tert.-Butyldimethylsilyloxymethyl)-(1RS)-cyclohex-1-yl]-(1E, 3E)-(5RS)-trideca-1,3-dien-7-in-5-ol-tert.-butyldiphenylsilylether

11,7 g des vorstehend hergestellten Silylethers in 100 ml Tetrahydrofuran versetzt man bei -70°C unter Argon mit 19 ml einer 1,6 molaren n-Butyllithiumlösung in Hexan. Man läßt auf - 10°C erwärmen und rührt bei dieser Temperatur 30 Minuten. Anschließend gibt man nacheinander 10 ml n-Pentylbromid, 16 ml Hexamethylphosphorsäuretriamid und 400 mg Natriumjodid zu und rührt 6 Stunden bei 24°C. Man gibt das Reaktionsgemisch auf Wasser, extrahiert mehrmals mit Ether, trocknet über Natriumsulfat und dampft im Vakuum ein. Den Rückstand chromatographiert man an Kieselgel mit Hexan/Methylenchlorid (8+2). Man erhalt so 7,6 g der Titelverbindung als farbloses Öl.
- IR:: 2928, 2860, 1122, 990, 838, 700 cm⁻¹

### d) cis-(1RS)-1-Hydroxymethyl-(2RS)-[(1E,3E)-(5RS)-5-tert.-butyldiphenylsilyloxy-trideca-1,3-dien-7-in]-cyclohexan

Zu 7,6 g des vorstehend hergestellten Silylethers gelost in 100 ml Tetrahydrofuran gibt man 100 ml eines Gemisches von Essigsäure/Wasser/THF (65:35:10) und rührt 10 Stunden auf 50°C. Unter Toluolzusatz engt man im Vakuum ein. Den Rückstand chromatographiert man an Kieselgel mit Hexan/0-40% Essigester. Man erhalt so 5,7 g der Titelverbindung als farbloses Öl.
- IR:: 3580, 3350, 3315, 2930, 2860, 1122, 990, 702 cm⁻¹

### e) cis-(1RS)-1-Formyl-(2RS)-[(1E,3E)-(5RS)-5-tert.-butyldiphenylsilyl-oxy-trideca-1,3-dien-7-in]-cyclohexan

1,0 g des vorstehend hergestellten Alkohols in 80 ml Methylenchlorid versetzt man bei 0°C unter Argon mit 6 g Collins-Reagenz (Chromsäure-Pyridin-Komplex) und rührt 30 Minuten bei 0°C. Anschließend filtriert man uber Celite ab, wäscht mit Ether nach und dampft im Vakuum ein. Der so erhaltene Aldehyd wird ohne weitere Reinigung verwendet.

### BEISPIEL 60

90 mg (±)-(5RS)-5-Acetoxy-5-[cis-(2RS)-2-((1E,3E)-(5RS)-5-tert.-butyldiphenylsilyloxy-trideca-1,3-dien-7-inyl)-(1RS)-cyclohex-1-yl]-pentansäure (hergestellt in Beispiel 59) wird zusammen mit 1,8 ml ethanolische Kaliumhydroxid-Losung (5 g Kaliumhydroxid in 62,5 ml Wasser und 187,5 ml Ethanol) 4 Tage bei 24°C gerührt. Anschl ießend wird mit 10%iger Schwefelsäure auf pH=5 angesäuert, mehrmals mit Essigester extrahiert, die organische Phase mit Sole gewaschen, über Natriumsulfat getrocknet und im Vakuum eingedampft. Den Rückstand chromatographiert man an Kieselgel mit Hexan/10-70% Essigester. Man erhält 32 mg (±)-(5RS)-5-Acetoxy-5-[cis-(2RS)-2-((1E,3E)-(5RS)-5-hydroxy-trideca-1,3-dien-7-inyl)-(1RS)-cyclohex-1-yl]-pentansäure.
- IR:: 3600, 3420, 2928, 2860, 1725, 1250, 1122, 990, 705 cm⁻¹
32 mg des vorstehend hergestellten Alkohols gelöst in 1,5 ml Tetrahydrofuran versetzt man bei 24°C unter Argon mit 180 mg Tetrabutylammoniumfluorid und rührt 16 Stunden bei dieser Temperatur. Anschließend verdünnt man mit Ether, wascht die organische Phase mit Sole, trocknet über Natriumsulfat und dampft im Vakuum ein. Den Rückstand chromatographiert man an Kieselgel mit Hexan/30-70% Essigester. Man erhält 6,5 mg der Titelverbindung als farbloses Öl.
- IR:: 3600, 3410, 2930, 2862, 1728, 1250, 1120, 990 cm⁻¹

### BEISPIEL 61

### (±)-((5RS)-5-Acetoxy-5-[cis-(2RS)-2-((1E,3E,7Z)-(5RS)-5-hydroxy-1,3,7--tridecatrienyl)-(1RS)-cyclohex-1-yl]-pentansäure Diastereomeres A und B

Zu 960 mg Magnesium tropft man bei 25°C unter Argon eine Lösung von 4,44 g 4-Chlor-1-(tert.butyldimethylsilyloxy)-butan in 4 ml Tetrahydrofuran und 0,06 ml Dibro methan, erwärmt 10 Minuten auf 60°C, rührt 30 Minuten bei 25°C und verdünnt mit 12,4 ml Tetrahydrofuran. Zu 1,95 g cis-(1RS)-1-Formyl-(2RS)-[(1E,3E,-7Z)-(5RS)-5-tert.-butyldiphenylsilyloxy-1,3,7-tridecatrienyl]-cyclohexan gelöst in 10 ml Tetrahydrofuran gibt man bei -70°C unter Argon 9 ml der oben hergestellten Grignard-Lösung und rührt 1 Stunde bei -70°C. Die Reaktionsmischung gibt man auf gesättigte Ammoniumchlorid-Lösung, extrahiert mehrmals mit Ether, wäscht die organische Phase mit Sole, trocknet über Natriumsulfat und dampft im Vakuum ein. Den so erhaltenen Rückstand löst man in 8 ml Pyridin, gibt unter Argon 2 ml Essigsaureanhydrid zu und rührt 20 Stunden bei 24°C. Anschließend dampft man im Vakuum unter Zusatz von Toluol ein. Den Rückstand chromatographiert man an Kieselgel mit Hexan/Ether (98+2). Man erhält 602 mg eines unpolaren und 621 mg eines polaren Diastereomeren von (±)-(5RS)-5-Acetoxy-5-[cis-(2RS)-2-((1E,3E,7Z)-(5RS)-5-tert.-butyldiphenylsilyloxy-1,3,7-tridecatrienyl)-(1RS)-cyclohex-1-yl]-pentan-1-ol-tert.-butyldimethylsilylether als farblose Öle.
- IR (unpolares Diastereomeres):: 2928, 2858, 1738, 1245, 988, 837, 703 cm⁻¹
- IR (polares Diastereomeres):: 2928, 2857, 1738, 1243, 990, 835, 702 cm⁻¹
Zu einer Losung von 240 mg des vorstehend hergestellten unpolaren Acetats in 12 ml Methanol gibt man bei 24°C unter Argon 2,4 ml einer HF-Pyridin-Losung (1 ml HF-Pyridin-Komplex + 3 ml Pyridin + 3 ml Tetrahydrofuran) und rührt 6 Stunden bei 24°C. Man verdünnt mit Ether, wäscht die organische Phase mit gesättigter Natriumhydrogencarbnnat-Lösung, mit Sole, trocknet über Natriumsulfat, filtriert und dampft im Vakuum ein. Den Rückstand chromatographiert man an Kieselgel mit Hexan/0-50% Essigester. Man erhält 97 mg (±)-(5RS)-5-Acetoxy-5-[cis-(2RS)-2-((1E,3E,7Z)-(5RS)-5-tert.-butyldiphenylsilyloxy-1,3,7-tridecatrienyl)-(1RS)-cyclohex-1-yl]-pentan-1-ol (Diastereomeres A) als farbloses Öl.
- IR (Diastereomeres A): 3450, 2930, 2860, 1735, 1242, 988, 702 cm⁻¹
In Analogie zur vorstehend beschriebenen Methode erhält man aus 621 mg des zuvor hergestellten polaren Acetats 169 mg des Distereomeren B als farbloses Öl.
- IR (Diastereomeres B): 3440, 2929, 2858, 1738, 1242, 990, 702 cm⁻¹
97 mg des vorstehend hergestellten Alkohols (Diastereomeres A) in 5 ml Methylenchlorid versetzt man bei 0°C unter Argon mit 400 mg Collins-Reagenz (Chromsäure-Pyridin-Komplex) und rührt 30 Minuten bei 0°C. Man verdünnt mit Hexan/Ether (1+1), filtriert über Celite ab, wäscht mit Hexan/Ether (1+1) nach und dampft im Vakuum ein. Den so erhaltenen Rückstand löst man in 4 ml Aceton, tropft unter Rühren bei -20°C 94 ml Jones-Reagenz (J.Chem.Soc. 1953, 2555) und rührt 25 Minuten bei -20°C unter Argon. Anschließend fügt man 0,1 ml Isopropanol zu, rührt 10 Minuten bei -20°C, verdünnt mit Ether, wäscht mit Sole/Wasser (1+1) neutral, trocknet über Natriumsulfat und dampft im Vakuum ein. Den Rückstand chromatographiert man an Kieselgel mit Hexan/0-30% Essigester. Man erhält 65 mg (±)-(5RS)-5-Acetoxy-5-[cis-(2RS)-2-((1E,3E,7Z)-(5RS)-5-tert.-butyldiphenylsilyloxy-1,3,7-tridecatrienyl)-(1RS)-cyclohex-1-yl]-pentansäure (Diastereomeres A) als farbloses Öl.
- IR (Diastereomeres A):: 3440, 3200, 2929, 2858, 1735, 1710, 1240, 990, 702 cm⁻¹
In Analogie zur vorstehend beschriebenen Methodik erhält man aus 261 mg des zuvor hergestellten Alkohols (Diastereomeres A) 162 mg der Säure (Diastereomeres B) als farbloses Öl.
- IR (Diastereomeres B):: 3450, 3200, 2930, 2858, 1738, 1708, 1240, 988, 702 cm⁻¹
65 mg der vorstehend hergestellten Saure (Diastereomeres A) gelöst in 2,7 ml Tetrahydrofuran versetzt man mit 366 mg Tetrabutylammoniumfluorid und rührt 16 Stunden bei 24°C unter Argon. Anschließend verdünnt man mit Ether, wäscht mit Sole, trocknet über Natriumsulfat und dampft im Vakuum ein. Den Rückstand chromatographiert man an Kieselgel mit Hexan/50-70% Essigester. Man erhält 24 mg der Titelverbindung (Diastereomeres A) als farbloses Öl.
- IR (Diastereomes A):: 3450, 3200, 2930, 2858, 1735, 1710, 1240, 988, 702 cm⁻¹
In Analogie zur vorstehend beschriebenen Methodik erhält man aus 160 mg der zuvor hergestellten Saure (Diastereomeres B) 64 mg der Titelverbindung (Diastereomeres B) als farbloses Öl.
- IR (Diastereomeres B):: 3440, 2928, 2857, 1710, 1242, 990 cm⁻¹
Das Ausgangsmaterial für die obige Titelverbindung wird wie folgt hergestellt:

### a) cis-(1RS)-1-Hydroxymethyl-(2RS)-[(1E,3E,7Z)-(5RS)-5-tert.-butyldiphenylsilyloxy-1,3,7-tridecatrienyl]-cyclohexan

Zur Hydrierung rührt man 6,2 g cis-(1RS)-1-Hydroxymethyl-(2RS)-[(1E,3E)-(5RS)-5-tert.-butyldiphenylsilyloxy-trideca-1,3-dien-7-in]-cyclohexan in 2,5 l Hexan/Essigester (1+1) mit 6,2 g Lindlar-Katalysator für 3 Stunden bei 24°C in einer Wasserstoffatmosphäre. Anschließend wird mit Stickstoff gespült. filtriert und im Vakuum eingeengt. Den Rückstand chromatographiert man an Kieselgel mit Hexan/tert.-Butylmethylether (9+1). Man erhält 3,43 g der Titelverbindung als farbloses Öl.
- IR:: 3590, 3340, 2925, 2855, 988, 698 cm⁻¹

### b) cis-(1RS)-1-Formyl-(2RS)-[(1E,3E,7Z)-(5RS)-5-tert.-butyldiphenylsilyloxy-1,3,7-tridecatrienyl]-cyclohexan

In Analogie zu Beispiel 59e erhält man aus 2,0 g des vorstehend hergestellten Alkohols 1,9 g der Titelverbindung als farbloses Öl.
- IR:: 2930, 2858, 2720, 1718, 990, 700 cm⁻¹

### BEISPIEL 62

### (±)-(5RS)-5-Hydroxy-5-[cis-(2RS)-2-((1E,3E,7Z)-(5RS)-5-hydroxy-1,3,7-tridecatrienyl)-(1RS)-cyclohex-1-yl]-pentansäure Diastereomeres A

20 mg (±)-(5RS)-5-Acetoxy-5-[cis-(2RS)-2-((1E,3E,7Z)-(5RS)-5-hydroxy -1,3,7-tridecatrienyl)-(1RS)-cyclohex-1-yl]-pentansäure (Diastereomeres A, hergestellt in Beispiel 61) gelöst in 0,6 ml Methanol versetzt man mit 0,6 ml 0.5 N Lithiumhydroxid-Lösung und rührt 24 Stunden bei 50°C unter Argon. Anschließend verdünnt man mit 1 ml Wasser und säuert bei 0°C mit einer 1 N Schwefelsäure auf pH=5 an. Man extrahiert mehrmals mit Wasser, wäscht die organische Phase mit Sole, trocknet über Natriumsulfat und dampft im Vakuum ein.

Den Rückstand chromatographiert man an Kieselgel mit Hexan/50-90% Essigester. Man erhält 11,2 mg der Titelverbindung als farbloses Öl.
- IR:: 3445, 2932, 2860, 1708, 1240, 990 cm⁻¹

### BEISPIEL 63

### (±)-(5RS)-5-Hydroxy-5-[cis-(2RS)-2-((1E,3E,7Z)-(5RS)-5-hydroxy-1,3,7-tridecatrienyl-(1RS)-cyclohex-1-yl]-pentansäure (Diastereomeres B)

In Analogie zu Beispiel 62 erhalt man aus 48 mg der nach Beispiel 61 hergestellten Saure (Diastereomeres B) 19 mg der Titelverbindung als farbloses Öl.
- IR:: 3440, 2930, 2858, 1710, 1240, 990 cm⁻¹

### BEISPIEL 64

### (±)-(5RS)-5-Acetoxy-5-[cis-(2RS)-2-((1E,3E)-(5RS)-5-acetoxy-6-(2-pentylphenyl) 1,3-hexadienyl)-(1RS)-cyclohex-1-yl]-pentan-1-ol Diastereomeres A

Zu 480 mg Magnesium tropft man bei 25°C unter Argon eine Losung von 2,2 g 4-Chlor-1-(tert.-butyldimethylsilyloxy)-butan in 2 ml Tetrahydrofuran und 0,06 ml Dibromethan, erwärmt 10 Minuten auf 60°C, rührt 30 Minuten bei 25°C und verdünnt mit 6,2 ml Tetrahydrofuran.
Zu 670 mg cis-(1RS)-1-Formyl-(2RS)-[1E,3E)-(5RS)-5-acetoxy-6-(2-pentylphenyl)-1,3-hexadienyl]-cyclohexan (Diastereomeres A) in 5 ml Tetrahydrofuran gibt man bei -70°C unter Argon 3 ml der oben hergestellten Grignard-Lösung und rührt 1 Stunde bei -70°C. Die Reaktionsmischung gibt man auf gesättigte Ammoniumchlorid-Lösung, extrahiert mehrmals mit Ether, wäscht die organische Phase mit Sole, trocknet über Natriumsulfat und dampft im Vakuum ein. Den so erhaltenen Rückstand löst man in 8 ml Pyridin, gibt unter Argon 2 ml Essigsäureanhydrid zu und rührt 24 Stunden bei 24°C. Anschließend dampft man im Vakuum unter Zugabe von Toluol ein. Den Rückstand chromatographiert man an Kieselgel mit Hexan/0-10% Essigester. Man erhalt 304 mg (±)-(5RS)-5-Acetoxy-5-[cis-(2RS)-2-((1E,3E)-(5RS)-5-acetoxy-6-(2-pentylphenyl)-1,3-hexadienyl)-(1RS)-cyclohex-1-yl]- pentan-1-ol-tert.-butyldimethylsilylether (Diastereomeres A).
- IR:: 2940, 2870, 1728, 1610, 1255, 972, 842 cm⁻¹
Zur Schutzgruppenspaltung werden 290 mg des vorstehend hergestellten Acetats gelöst in 29 ml Tetrahydrofuran mit 438 mg Tetrabutylammoniumfluorid versetzt und 4 Stunden bei 24°C unter Argon gerührt. Anschließend verdünnt man mit Ether, wäscht die organische Phase zweimal mit Wasser, trocknet über Magnesiumsulfat und dampft im Vakuum ein. Den Rückstand chromatographiert man an Kieselgel mit Hexan/0-70% Ether. Man erhält 188 mg der Titelverbindung als farbloses Öl.
- IR:: 3700, 3640, 3500, 2940, 2870, 1730, 1610, 1250, 972 cm⁻¹
Das Ausgangsmaterial für die obige Titelverbindung wird wie folgt hergestellt:

### a) 2-Pentylbenzylalkohol

Zu einer Lösung von 20,0 g 2-Methylbenzylalkohol in 185 ml Ether tropft man unter Argon 245 ml einer 1,6 molaren Butyllithium-Lösung in Hexan zu, so daß die Temperatur nicht über 10°C steigt. Anschließend erhitzt man 5 Stunden unter Rückfluß, läßt auf 24°C abkühlen, tropft 22,4 g 1-Brombutan zu und rührt 16 Stunden bei 24°C. Man gibt die Reaktionsmischung auf 100 ml Wasser und extrahiert dreimal mit je 100 ml Ether. Die organische Phase wascht man mit Sole, trocknet über Natriumsulfat und dampft im Vakuum ein. Den Rückstand chromatographiert man an Kieselgel mit Hexan/0-30% Essigester. Man erhalt 20,1 g der Titelverbindung als farbloses Öl.
- IR:: 3540, 3320, 2930, 2860, 1605, 750 cm⁻¹

### b) 2-Pentylbenzylbromid

Zu einer Lösung von 21 g des vorstehend hergestellten Alkohols in 23,8 ml Ether gibt man 3,2 ml Pyridin und tropft bei -10°C unter Argon 5,1 ml Phosphortribromid zu. Man erhitzt 3 Stunden unter Rückfluß und gibt die Reaktionsmischung nach dem Abkühlen auf 24°C vorsichtig auf 40 ml Wasser. Man extrahiert dreimal mit je 150 ml Ether, wäscht die organische Phase mit Sole, trocknet über Natriumsulfat und dampft im Vakuum ein. Den Rückstand chromatographiert man an Kieselgel mit Hexan/0-6% Ether. Man erhält 19,5 g der Titelverbindung als farbloses Öl.
- IR:: 2958, 2930, 2860, 1608, 760 cm⁻¹

### c) (5RS)-5-Acetoxy-1-[cis-(2RS)-2-tert.-butyldimethylsilyloxymethyl)-(1RS)-cyclohex-1-yl]-6-2-pentylphenyl)-1,3-hexadien

In Analogie zu Bei spiel 1c er hält man aus 5,7 g des nach Beispiel 12b hergestellten Aldehyds 4,1 g der Titelverbindung als farbloses Öl.
- IR:: 2930, 2858, 1735, 1605, 1240, 990, 845, 750 cm⁻¹

### d) cis-(1RS)-1-Formyl-(2RS)-[(1E,3E)-(5RS)-5-acetoxy-6-(2-pentylphenyl)-1,3-hexadienyl]-cyclohexan Diastereomeres A und Diastereomeres B

Zu einer Losung vonm 3,8 g des vorstehend hergestellten Acetats in 90 ml Tetrahydrofuran gibt man bei 0°C unter Argon 4,68 g Tetrabutylammoniumfluorid und rührt zunächst 30 Minuten bei 0°C, dann 4,5 Stunden bei 24°C. Man verdünnt mit Ether, wäscht die organische Phase zweimal mit Sole/Wasser (1+1), trocknet über Natriumsulfat und dampft im Vakuum ein. Den Rückstand chromatographiert man an Kieselgel mit Hexan/0-30% Essigester. Dabei erhält man zunächst 690 mg des unpolaren Diastereomeren und dann 1,05 g des polaren Diastereomeren cis-(1RS)-1-Hydroxymethyl-(2RS)-[(1E,3E)-(5RS)-5-acetoxy-6-(2-pentylphenyl)-1,3-hexadienyl]-cyclohexan als farblose Öle.
- IR (unpolares Diastereomeres):: 3440, 2925, 2857, 1738, 1605, 1235, 990, 750 cm⁻¹
- IR (polares Diastereomeres):: 3450, 2925, 2855, 1738, 1605, 1235, 990, 750 cm⁻¹
685 mg des vorstehend hergestellten Alkohols (unpolares Diastereomeres) gelöst in 17 ml Methylenchlorid versetzt man bei 0°C unter Argon mit 3,5 g Collins-Reagenz und rührt 20 Minuten bei 0°C. Man verdünnt mit Hexan/Ether (2+1), filtriert über Celite ab, wäscht mit Hexan/Ether (2+1) nach und dampft im Vakuum ein. Der so erhaltene Aldehyd (Diastereomeres A) wird ohne weitere Reinigung verwendet.

In Analogie zur vorstehend beschriebenen Methode erhält man aus 1,05 g des zuvor hergestellten Alkohols (polares Diastereomeres) den Aldehyd (Diastereomeres B).

### BEISPIEL 65

### (±)-(5RS)-5-Acetoxy-5-[cis-(2RS)-2-((1E,3E)-(5RS)-5-acetoxy-6-(2-pentylphenyl)-1,3-hexadienyl-(1RS)-cyclohex-1-yl]-pentan-1-ol

### Diastereomeres B

In Analogie zu Beispiel 64 erhalt man aus 980 mg des nach Beispiel 64d hergestellten Aldehyds (Diastereomeres B) 252 mg der Titelverbindung als farbloses Öl.
- IR:: 3700, 3620, 3480, 2938, 2870, 1732, 1605, 1250, 992 cm⁻¹

### BEISPIEL 66

### (±)-(5RS)-5-Acetoxy-5-[cis-(2RS)-2-((1E,3E)-(5RS)-5-acetoxy-6-(2-pentylphenyl)-1,3-hexadienyl)-(1RS)-cyclohex-1-yl]-pentansäure

### Diastereomeres A1 und Diastereomeres A2

185 mg des nach Beispiel 64 hergestellten Alkohols in 20 ml Methylenchlorid versetzt man bei 0°C unter Argon mit 1,1 g Collins-Reagenz (Chromsaure-Pyridin-Komplex) und rührt 30 Minuten bei 0°C. Man verdünnt mit Hexan/Ether (1+1), filtriert über Celite ab, wascht mit Hexan/Ether (1+1) nach und dampft im Vakuum ein. Den Rückstand löst man in 15 ml Aceton, tropft bei -30°C unter Rühren 0,35 ml Jones-Reagenz (J.Chem.Soc. 1953, 2555) und rührt 20 Minuten bei -20°C. Anschließend fügt man 0,5 ml Isopropanol zu, rührt 10 Minuten bei -20°C, verdünnt mit Ether, wäscht mit Sole/Wasser (1+1) neutral, trocknet über Natriumsulfat und dampft im Vakuum ein. Den Rückstand chromatographiert man an Kieselgel mit Hexan/0-60% Essigester. Man erhalt zunächst 75 mg des unpolaren Diastereomeren (Diastereomeres A1) und dann 50 mg des polaren Diastereomeren (Diastereomeres A2) als farblose Öle.
- IR (Diastereomeres A1):: 3520, 3180, 2937, 2860, 1730, 1605, 1250, 990 cm⁻¹
- IR (Diastereomeres A2):: 3520, 3100, 2937, 2860, 1730, 1605, 1248, 992 cm⁻¹

### BEISPIEL 67

### (±)-(5RS)-5-Acetoxy-5-[cis-(2RS)-2-((1E,3E)-(5RS)-5-acetoxy-6-(2-pentylphenyl)-1,3-hexadienyl)-(1RS)-cyclohex-1-yl]-pentansäure

### Diastereomeres B1 und Diastereomeres B2

In Analogie zu Beispiel 66 erhält man aus 240 mg des nach Beispiel 65 hergestellten Alkohols 95 mg des unpolaren Diastereomeren (Diastereomeres B1) und 60 mg des polaren Diastereomeren (Diastereomeres B2) der Titelverbindung als farblose Öle.
- IR (Diastereomeres B1):: 3520, 3150, 2935, 2860, 1730, 1605, 1250, 992 cm⁻¹
- IR (Diastereomeres B2):: 3520, 3100, 2937, 2862, 1730, 1605, 1248, 992 cm⁻¹

### BEISPIEL 68

### (±)-(5RS)-5-Acetoxy-5-[cis-(2RS(-2-((1E,3E)-(5RS)-5-hydroxy-6-(2-pentylphenyl)-1,3-hexadienyl)-(1RS)-cyclohex-1-yl]-pentansäure

### Diastereomeres A1

In Analogie zu Beispiel 4 erhält man aus 70 mg des nach Beispiel 66 hergestellten Diacetats (Diastereomeres A1) 35,4 mg der Titelverbindung als farbloses Öl.
- IR:: 3680, 3600, 3410, 2930, 2860, 1725, 1605, 1255, 992 cm⁻¹

### BEISPIEL 69

### (±)-(5RS)-5-Acetoxy-5-[cis-(2RS)-2-((1E,3E)-(5RS)-5-hydroxy-6-(2-pentylphenyl)-1,3-hexadienyl)-(1RS)-cyclohex-1-yl]-pentansäure

### Diastereomeres B1

In Analogie zu Beispiel 4 erhält man aus 90 mg des nach Beispiel 67 hergestellten Diacetats (Diastereomeres B1 55 mg der Titelverbindung als farbloses Öl.
- IR:: 3690, 3600, 3400, 2930, 2860, 1725, 1603, 1255, 993 cm⁻¹

### BEISPIEL 70

### (±)-(5RS)-5-Acetoxy-5-[cis-(2RS)-2-((1E,3E)-(5RS)-5-hydroxy-6-(2-pentylphenyl)-1,3-hexadienyl)-(1RS)-cyclohex-1-yl]-pentansäure

### Diastereomeres A2

In Analogie zu Beispiel 4 erhält man aus 45 mg des nach Beispiel 66 hergestellten Diacetats (Diastereomeres A2) 16,4 mg der Titelverbindung als farbloses Öl.
- IR:: 3600, 3520, 3420, 2930, 2860, 1730, 1605, 1250, 992 cm⁻¹

### BEISPIEL 71

### (±)-(5RS)-5-Acetoxy-5-[cis-(2RS)-2-((1E,3E)-(5RS)-5-hydroxy-6-(2-pentylphenyl)-1,3-hexadienyl)-(1RS)-cyclohex-1-yl]-pentansäure

### Diastereomeres B2

In Analogie zu Beispiel 4 erhalt man aus 55 mg des nach Beispiel 67 hergestellten Diacetats (Diastereomeres B2) 22,4 mg der Titelverbindung als farbloses Öl.
- IR:: 3600, 3520, 3400, 2935, 2860, 1730, 1605, 1255, 992 cm⁻¹

### BEISPIEL 72

### (±)-(5RS)-5-Hydroxy-5-[cis-(2RS)-2-((1E,3E)-(5RS)-5-hydroxy-6-(2-pentylphenyl)-1,3-hexadienyl)-(1RS)-cyclohex-1-yl]-pentansäure

### Diastereomeres A1

In Analogie zu Beispiel 62 erhält man aus 28 mg des nach Beispiel 68 hergestellten Monoacetats 14,8 mg der Titelverbindung als farbloses Öl.
- IR:: 3680, 3520, 2930, 2860, 1728, 1605, 1248, 992 cm⁻¹

### BEISPIEL 73

### (±)-(5RS)-5-Hydroxy-5-[cis-(2RS)-2-((1E,3E)-(5RS)-5-hydroxy-6-(2-pentylphenyl)-1,3-hexadienyl)-(1RS)-cyclohex-1-yl]-pentansäure

### Diastereomeres B1

In Analogie zu Beispiel 62 erhält man aus 38 mg des nach Beispiel 69 hergestellten Monoacetats 15,2 mg der Titelverbindung als farbloses Öl.
- IR:: 3680, 3510, 2935, 2860, 1725, 1605, 1250, 992 cm⁻¹

### BEISPIEL 74

### (±)-(5RS)-5-Hydroxy-5-[cis-(2RS)-2-((1E,3E)-(5RS)-5-hydroxy-6-(2-pentylphenyl)-1,3-hexadienyl)-(1RS)-cyclohex-1-yl]-pentansäure

### Diastereomeres A2

In Analogie zu Beispiel 62 erhält man aus 13 mg des nach Beispiel 70 hergestellten Monoacetats 3,8 mg der Titelverbindung als farbloses Öl.
- IR:: 3690, 3610, 3440, 2935, 2862, 1722, 1605, 1260, 993 cm⁻¹

### BEISPIEL 75

### (±)-(5RS)-5-Hydroxy-5-[cis-(2RS)-2-((1E,3E)-(5RS)-5-hydroxy-6-(2-pentylphenyl)-1,3-hexadienyl)-(1RS)-cyclohex-1-yl]-pentansäure

### Diastereomeres B2

In Analogie zu Beispiel 62 erhalt man aus 18 mg des nach Beispiel 71 hergestellten Monoacetats 7,4 mg der Titelverbindung als farbloses Öl.
- IR:: 3680, 3520, 3410, 2930, 2860, 1720, 1605, 1245, 992 cm⁻¹

## Patentansprüche

1. Verbindungen der Formel I, 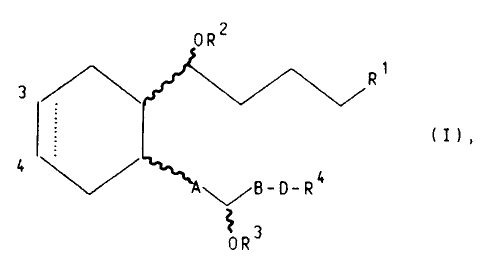 wobei die Reste folgende Bedeutungen haben: 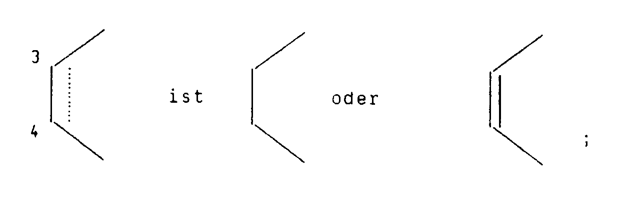
R¹ ist CH₂OH, CH₃, CF₃, COOR⁵ mit R⁵ in der Bedeutung eines Wasserstoffatoms, eines Alkylrestes mit 1-10 C-Atomen, eines Cycloalkylrestes mit 3-10 C-Atomen, eines gegebenenfalls durch 1-3 Chlor, Brom, Phenyl, C₁₋₄-Alkyl, C₁₋₄-Alkoxy, Chlormethyl, Fluormethyl, Trifluormethyl, Carboxy oder Hydroxy substituierten Arylrestes mit 6-10 C-Atomen, eines -CH₂-CO-Aryl-Restes mit 6-10 C-Atomen für Aryl oder eines 5-6-gliedrigen aromatischen heterocyclischen Restes mit wenigstens 1 Heteroatom oder
R¹ ist CONHR⁶ mit R⁶ in der Bedeutung eines Alkanoyl- oder Alkansulfonylrestes mit 1-10 C-Atomen oder des Restes R⁵;
A ist eine trans, trans-CH=CH-CH=CH- oder Tetramethylengruppe;
B ist eine geradkettige oder verzweigtkettige gesättigte oder ungesättigte Alkylengruppe mit bis zu 10 C-Atomen, die gegebenenfalls durch Fluor substituiert sein kann, oder die Gruppe 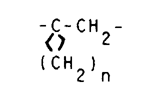 mit n=1-3;
D ist eine Direktbindung, Sauerstoff, Schwefel, eine -C≡C-Gruppe oder eine -CH=CR⁷-Gruppe mit R⁷ als Wasserstoff, C₁₋₅-Alkyl, Chlor, Brom oder 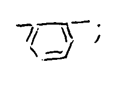
B und D sind gemeinsam, eine Direktbindung;
R² und R³ sind gleich oder verschieden und bedeuten Wasserstoff oder einen organischen Säurerest mit 1-15 C-Atomen;
R¹ und R² sind gemeinsam eine Carbonylgruppe;
R⁴ ist ein Wasserstoffatom, C₁₋₁₀Alkyl, das gegebenenfalls durch Chlor oder Brom substituiert sein kann, Cycloalkyl mit 3-10 C-Atomen , ein gegebenenfalls durch 1-3 Chlor, Brom, Phenyl C₁₋₄-Alkyl, C₁₋₄-Alkoxy, Chlormethyl, Fluormethyl, Trifluormethyl, Carboxy oder Hydroxy substituierter Arylrest mit 6-10 C-Atomen oder ein 5-6-gliedriger aromatischer heterocyclischer Rest mit wenigstens 1-Heteroatom und falls
R⁵ ein Wasserstoffatom bedeutet, deren Salze mit physiologisch verträglichen Basen und deren Cyclodextrinclathrate.

2. Verbindungen der Formel I gemäß Anspruch 1, wobei die Reste die folgende Bedeutung haben: 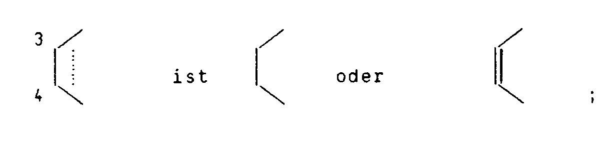
R¹ ist CH₂OH, COOR⁵ mit R⁵ in der Bedeutung eines Wasserstoffatoms, eines Alkylrestes mit 1-10 C-Atomen, eines Cycloalkylrestes mit 5-6 C-Atomen, eines gegebenenfalls durch 1-2 Chlor, Brom, Phenyl, C₁₋₄-Alkyl, C₁₋₄-Alkoxy, Chlormethyl, Fluormethyl, Trifluormethyl, Carboxy oder Hydroxy substituierten Phenylrestes oder
R¹ ist CONHR⁶ mit R⁶ in der Bedeutung eines Alkanoyl- oder Alkansulfonylrestes mit 1-10 C-Atomen oder des Restes R⁵;
A ist eine trans, trans-CH=CH-CH=CH- oder Tetramethylengruppe;
B ist eine geradkettige oder verzweigtkettige gesättigte oder ungesättigte Alkylengruppe mit bis zu 10 C-Atomen, die gegebenenfalls durch Fluor substituiert sein kann, oder die Gruppe 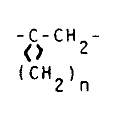 mit n=1-3;
D ist eine Direktbindung, Sauerstoff, Schwefel, eine -C≡C-Gruppe oder eine -CH=CR⁷-Gruppe mit R⁷ als Wasserstoff, C₁₋₅-Alkyl, Chlor, Brom oder 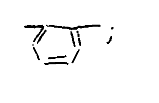
B und D sind gemeinsam eine Direktbindung;
R² und R³ sind gleich oder verschieden und bedeuten Wasserstoff oder einen organischen Säurerest mit 1-15 C-Atomen;
R¹ und R² sind gemeinsam eine Carbonylgruppe;
R⁴ ist ein Wasserstoffatom, C₁₋₁₀-Alkyl, Cycloalkyl mit 5-6 C-Atomen , ein gegebenenfalls durch 1-2 Chlor, Brom, Phenyl, C₁₋₄-Alkyl, C₁₋₄-Alkoxy, Chlormethyl, Fluormethyl, Trifluormethyl, Carboxy oder Hydroxy substituierter Phenylrest und falls
R⁵ ein Wasserstoffatom bedeutet, deren Salze mit physiologisch verträglichen Basen und deren Cyclodextrinclathrate.

3. Verbindungen der Formel I gemäß Anspruch 1, wobei die Reste die folgende Bedeutung haben: 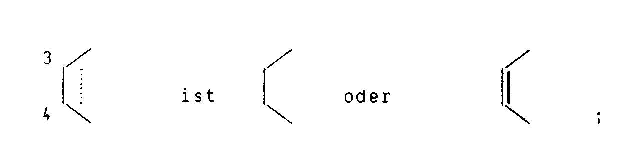
R¹ ist CH₂OH, COOR⁵ mit R⁵ in der Bedeutung eines Wasserstoffatoms, eines Alkylrestes mit 1-4 C-Atomen;
A ist eine trans, trans-CH=CH-CH=CH- oder Tetramethylengruppe;
B ist eine geradkettige oder verzweigtkettige Alkylengruppe mit bis zu 5 C-Atomen;
D ist eine Direktbindung oder eine -C≡C-Gruppe oder eine -CH=CR⁷-Gruppe mit R⁷ als Wasserstoff oder C₁₋₅-Alkyl; oder 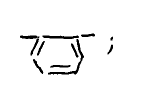
B und D sind gemeinsam eine Direktbindung;
R² und R³ sind gleich oder verschieden und bedeuten Wasserstoff oder einen organischen Säurerest mit 1-6 C-Atomen;
R¹ und R² sind gemeinsam eine Carbonylgruppe;
R⁴ ist ein Wasserstoffatom oder C₁₋₁₀-Alkyl und falls
R⁵ ein Wasserstoffatom bedeutet, deren Salze mit physiologisch verträglichen Basen und deren Cyclodextrinclathrate.

4. Verfahren zur Herstellung der erfindungsgemäßen Verbindungen der Formel I, dadurch gekennzeichnet, daß man einen Aldehyd der Formel II 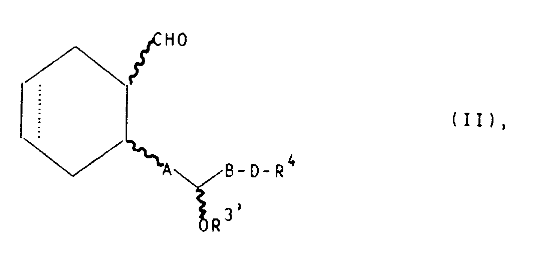 worin A, B, D und R⁴ die oben angegebene Bedeutung haben und R^{3'} Silylschutzgruppen bedeuten, gegebenenfalls nach Schutz freier Hydroxygruppen mit einer magnesiumorganischen Verbindung der Formel III,
X-Mg-CH₂-CH₂-CH₂-CH₂-O-R⁸ (III),
worin X Chlor, Brom oder Jod und R⁸ einen leicht abspaltbaren Etherrest bedeuten, umsetzt und gegebenenfalls anschließend in beliebiger Reihenfolge Isomere trennt, geschützte Hydroxygruppen freisetzt und/oder eine freie Hydroxygruppe verestert und/oder die 1-Hydroxygruppe zur Carbonsäure oxidiert und/oder Doppelbindungen hydriert und/oder eine veresterte Carboxylgruppe (R¹=COOR⁵) verseift und/oder reduziert und/oder eine Carboxylgruppe (R⁵=H) verestert und/oder eine freie Carboxylgruppe (R⁵=H) in ein Amid (R¹=CONHR⁶) überführt oder eine Carboxylgruppe mit einer physiologisch verträglichen Base in ein Salz überführt.

5. Pharmazeutische Zubereitungen, die eine oder mehrere der Verbindungen nach Anspruch 1 enthalten.

## Claims

1. Compounds of formula I 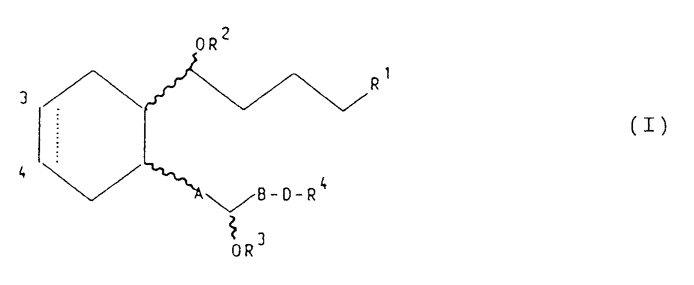 wherein the radicals have the following meanings: 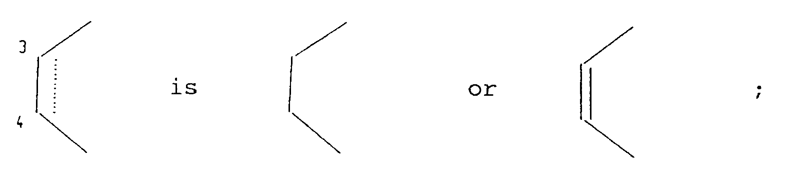
R¹ is CH₂OH, CH₃, CF₃, or COOR⁵ wherein R⁵ represents a hydrogen atom, an alkyl radical having from 1 to 10 carbon atoms, a cycloalkyl radical having from 3 to 10 carbon atoms, an aryl radical having from 6 to 10 carbon atoms that is optionally substituted by from 1 to 3 chlorine atoms, bromine atoms, phenyl groups, C₁₋₄-alkyl groups, C₁₋₄-alkoxy groups, chloromethyl groups, fluoromethyl groups, trifluoromethyl groups, carboxy groups or hydroxy groups, a -CH₂-CO-aryl radical having from 6 to 10 carbon atoms in the aryl moiety or a 5- or 6-membered aromatic heterocyclic radical having at least one hetero atom, or
R¹ is CONHR⁶ wherein R⁶ represents an alkanoyl or alkanesulphonyl radical having from 1 to 10 carbon atoms or a radical R⁵;
A is a trans, trans-CH=CH-CH=CH- or a tetramethylene group;
B is a straight-chain or branched-chain saturated or unsaturated alkylene group having up to 10 carbon atoms that may optionally be substituted by fluorine, or is the group 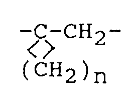 wherein n = 1 to 3;
D is a direct bond, oxygen, sulphur, a -C≡C- group or a -CH=CR⁷- group wherein R⁷ represents hydrogen, C₁₋₅-alkyl, chlorine or bromine; or 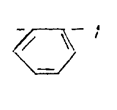
B and D together form a direct bond;
R² and R³ are the same or different and represent hydrogen or an organic acid radical having from 1 to 15 carbon atoms;
R¹ and R² together form a carbonyl group;
R⁴ is a hydrogen atom, C₁₋₁₀-alkyl which may optionally be substituted by chlorine or by bromine, cycloalkyl having from 3 to 10 carbon atoms, an aryl radical having from 6 to 10 carbon atoms that is optionally substituted by from 1 to 3 chlorine atoms, bromine atoms, phenyl groups, C₁₋₄-alkyl groups, C₁₋₄-alkoxy groups, chloromethyl groups, fluoromethyl groups, trifluoromethyl groups, carboxy groups or hydroxy groups, or a 5- or 6-membered aromatic heterocyclic radical having at least one hetero atom and if
R⁵ represents a hydrogen atom, the salts thereof with physiologically tolerable bases and the cyclodextrin clathrates thereof.

2. Compounds of formula I according to claim 1, wherein the radicals have the following meanings: 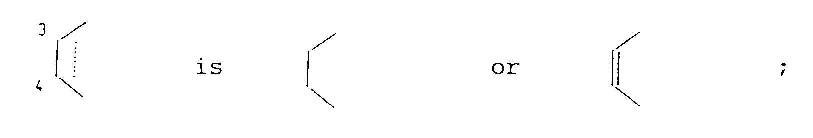
R¹ is CH₂OH, or COOR⁵ wherein R⁵ represents a hydrogen atom, an alkyl radical having from 1 to 10 carbon atoms, a cycloalkyl radical having 5 or 6 carbon atoms, a phenyl radical that is optionally substituted by 1 or 2 chlorine atoms, bromine atoms, phenyl groups, C₁₋₄-alkyl groups, C₁₋₄-alkoxy groups, chloromethyl groups, fluoromethyl groups, trifluoromethyl groups, carboxy groups or hydroxy groups, or
R¹ is CONHR⁶ wherein R⁶ represents an alkanoyl or alkanesulphonyl radical having from 1 to 10 carbon atoms or a radical R⁵;
A is a trans, trans-CH=CH-CH=CH- or a tetramethylene group;
B is a straight-chain or branched-chain saturated or unsaturated alkylene group having up to 10 carbon atoms that may optionally be substituted by fluorine, or is the group 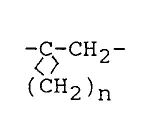 wherein n = 1 to 3;
D is a direct bond, oxygen, sulphur, a -C≡C- group or a -CH=CR⁷- group wherein R⁷ represents hydrogen, C₁₋₅-alkyl, chlorine or bromine; or 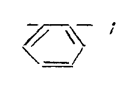
B and D together form a direct bond;
R² and R³ are the same or different and represent hydrogen or an organic acid radical having from 1 to 15 carbon atoms;
R¹ and R² together form a carbonyl group;
R⁴ is a hydrogen atom, C₁₋₁₀-alkyl, cycloalkyl having 5 or 6 carbon atoms, a phenyl radical that is optionally substituted by 1 or 2 chlorine atoms, bromine atoms, phenyl groups, C₁₋₄-alkyl groups, C₁₋₄-alkoxy groups, chloromethyl groups, fluoromethyl groups, trifluoromethyl groups, carboxy groups or hydroxy groups, and if
R⁵ represents a hydrogen atom, the salts thereof with physiologically tolerable bases and the cyclodextrin clathrates thereof.

3. Compounds of formula I according to claim 1, wherein the radicals have the following meanings: 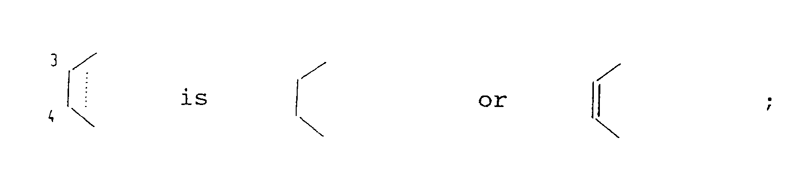
R¹ is CH₂OH, or COOR⁵ wherein R⁵ represents a hydrogen atom or an alkyl radical having from 1 to 4 carbon atoms;
A is a trans, trans-CH=CH-CH=CH- or a tetramethylene group;
B is a straight-chain or branched-chain alkylene group having up to 5 carbon atoms;
D is a direct bond, a -C≡C- group or a -CH=CR⁷- group wherein R⁷ represents hydrogen or C₁₋₅-alkyl; or 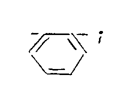
B and D together form a direct bond;
R² and R³ are the same or different and represent hydrogen or an organic acid radical having from 1 to 6 carbon atoms;
R¹ and R² together form a carbonyl group;
R⁴ is a hydrogen atom or C₁₋₁₀-alkyl, and if
R⁵ represents a hydrogen atom, the salts thereof with physiologically tolerable bases and the cyclodextrin clathrates thereof.

4. Process for the preparation of the compounds of formula I according to the invention, characterised in that an aldehyde of formula II 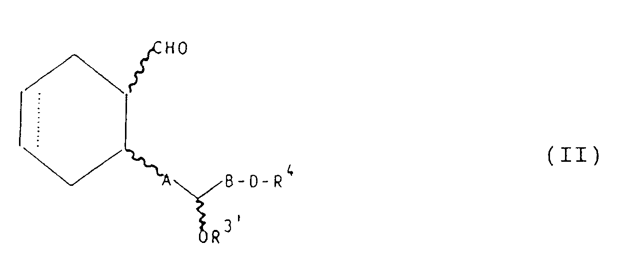 wherein A, B, D and R⁴ are as defined above and R^{3'} represents a silyl protecting group, is reacted, optionally after protecting free hydroxy groups, with an organomagnesium compound of formula III
X-Mg-CH₂-CH₂-CH₂-CH₂-O-R⁸ (III),
wherein X represents chlorine, bromine or iodine and R⁸ represents a readily removable ether radical, and then optionally, in any sequence, isomers are separated, protected hydroxy groups are freed and/or a free hydroxy group is esterified and/or the 1-hydroxy group is oxidised to the carboxylic acid and/or double bonds are hydrogenated and/or an esterified carboxy group (R¹ = COOR⁵) is hydrolysed and/or reduced and/or a carboxy group (R⁵ = H) is esterified and/or a free carboxy group (R⁵ = H) is converted into an amide (R¹ = CONHR⁶) or a carboxy group is converted into a salt with a physiologically tolerable base.

5. Pharmaceutical preparations that contain one or more of the compounds according to claim 1.

## Revendications

1. Composés de formule I 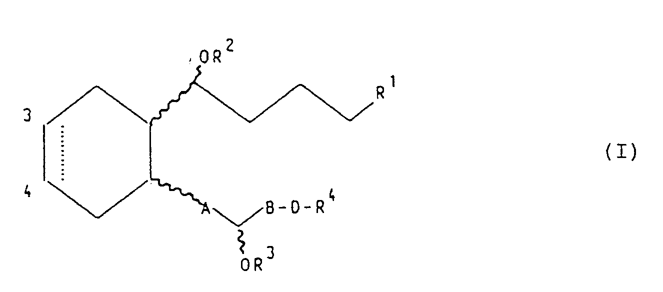 les radicaux ayant les significations suivantes : 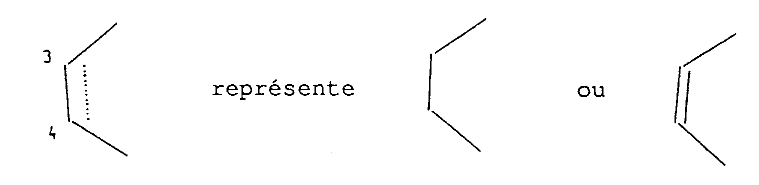
R¹ représente CH₂OH, CH₃, CF₃, COOR⁵ avec R⁵ ayant la signification d'un atome d'hydrogène, d'un radical alkyle comportant de 1 à 10 atomes de carbone, d'un radical cycloalkyle comportant de 3 à 10 atomes de carbone, d'un radical aryle comportant de 6 à 10 atomes de carbone éventuellement substitué par 1 à 3 chlore, brome, phényle, alkyle en C₁₋₄, alcoxy en C₁₋₄, chlorométhyle, fluorométhyle, trifluorométhyle, carboxy ou hydroxy, d'un radical -CH₂-CO-aryle comportant de 6 à 10 atomes de carbone pour l'aryle ou d'un radical hétérocyclique aromatique à 5 à 6 chaînons comportant au moins un hétéroatome, ou
R¹ représente CONHR⁶ avec R⁶ ayant la signification d'un radical alcanoyl- ou alcane-sulfonyle comportant de 1 à 10 atomes de carbone ou du radical R⁵;
A représente un trans, trans-CH=CH-CH=CH- ou un groupe tétraméthylène;
B représente un groupe alkylène linéaire ou ramifié, saturé ou insaturé, comportant jusqu'à 10 atomes de carbone, qui peut être éventuellement substitué par le fluor, ou représente le groupe 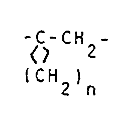 avec n = 1 à 3;
D est une liaison directe, l'oxygène, le soufre, un groupe -C≡C- ou un groupe -CH=CR⁷ avec R⁷ en tant qu'hydrogène, alkyle en C₁₋₅, chlore, brome ou 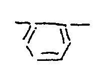
B et D ensemble, représentent une liaison directe;
R² et R³ sont identiques ou différents et signifient l'hydrogène ou un radical d'acide organique comportant de 1 à 15 atomes de carbone;
R¹ et R² ensemble, représentent un groupe carbonyle;
R⁴ représente un atome d'hydrogène, un alkyle en C₁₋₁₀, qui peut être éventuellement substitué par le chlore ou le brome, un cycloalkyle comportant de 3 à 10 atomes de carbone, un radical aryle comportant de 1 à 10 atomes de carbone éventuellement substitué par 1 à 3 chlore, brome, phényle, alkyle en C₁₋₄, alcoxy en C₁₋₄, chlorométhyle, fluorométhyle, trifluorométhyle, carboxy ou hydroxy, ou représente un radical hétérocyclique aromatique à 5 à 6 chaînons comportant au moins un hétéroatome, et si
R⁵ signifie un atome d'hydrogène, leurs sels avec des bases physiologiquement compatibles et leurs clathrates de cyclodextrine.

2. Composés de formule I selon la revendication 1, les radicaux ayant la signification suivante : 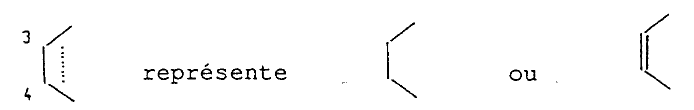
R¹ représente CH₂OH, COOR⁵ avec R⁵ ayant la signification d'un atome d'hydrogène, d'un radical alkyle comportant de 6 à 10 atomes de carbone, d'un radical cycloalkyle comportant de 5 à 6 atomes de carbone, d'un radical phényle éventuellement substitué par 1 à 2 chlore, brome, phényle, alkyle en C₁₋₄, alcoxy en C₁₋₄, chlorométhyle, fluorométhyle, trifluorométhyle, carboxy ou hydroxy, ou
R¹ représente CONHR⁶ avec R⁶ ayant la signification d'un radical alcanoyl- ou alcane-sulfonyle comportant de 1 à 10 atomes de carbone ou du radical R⁵ ;
A représente un trans,trans-CH=CH-CH=CH- ou un groupe tétraméthylène;
B représente un groupe alkylène linéaire ou ramifié saturé ou insaturé comportant jusqu'à 10 atomes de carbone, qui peut être éventuellement substitué par le fluor, ou représente le groupe 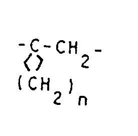 avec n = 1 à 3 ;
D représente une liaison directe, l'oxygène, le soufre, un groupe -C≡C- ou un groupe -CH=CR⁷ avec R⁷ en tant qu'hydrogène, alkyle en C₁₋₅, chlore, brome ou 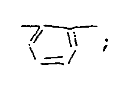
B et D ensemble, représentent une liaison directe ;
R² et R³ sont identiques ou différents et signifient l'hydrogène ou un radical d'acide organique comportant 1 à 15 atomes de carbone;
R¹ et R² ensemble, représentent un groupe carbonyle ;
R⁴ représente un atome d'hydrogène, les alkyle en C₁₋₁₀, cycloalkyle comportant 5 à 6 atomes de carbone, un radical phényle éventuellement substitué par 1 à 2 chlore, brome, phényle, alkyle en C₁₋₄, alcoxy en C₁₋₄, chlorométhyle, fluorométhyle, trifluorométhyle, carboxy ou hydroxy, et si
R⁵ signifie un atome d'hydrogène, leurs sels avec des bases physiologiquement compatibles et leurs clathrates de cyclodextrine.

3. Composés de formule I selon la revendication 1, les radicaux ayant la signification suivante : 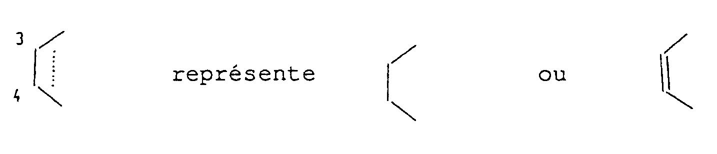
R¹ représente CH₂OH, COOR⁵ avec R⁵ ayant la signification d'un atome d'hydrogène, d'un radical alkyle comportant de 1 à 4 atomes de carbone;
A représente un trans,trans-CH=CH-CH=CH- ou un groupe tétraméthylène;
B représente un groupe alkylène linéaire ou ramifié comportant jusqu'à 5 atomes de carbone;
D représente une liaison directe ou un groupe -C≡C- ou un groupe -CH=CR⁷ avec R⁷ en tant qu'hydrogène ou un alkyle en C₁₋₅ ; ou 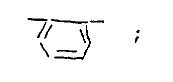
B et D ensemble, représentent une liaison directe ;
R² et R³ sont identiques ou différents et signifient l'hydrogène ou un radical d'acide organique comportant 1 à 6 atomes de carbone ;
R¹ et R² ensemble, représentent un groupe carbonyle ;
R⁴ représente un atome d'hydrogène ou un alkyle en C₁₋₁₀, et si
R⁵ signifie un atome d'hydrogène, leurs sels avec des bases physiologiquement compatibles et leurs clathrates de cyclodextrine.

4. Procédé pour la préparation des composés de formule I conformes à l'invention, caractérisé en ce qu'on fait réagir un aldéhyde de formule II 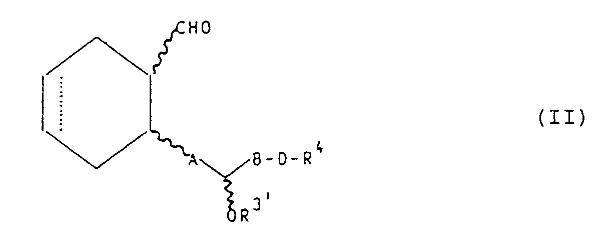 où A, B, D et R⁴ ont la signification donnée ci-dessus et R^{3'} signifie des groupes protecteurs de silyle, éventuellement après protection des groupes hydroxy libres, avec un composé organomagnésien de formule III
X-Mg-CH₂-CH₂-CH₂-CH₂-O-R⁸ (III)
où X signifie les chlore, brome ou iode et R⁸ un radical éther facilement éliminable, et éventuellement, on sépare ensuite, dans un ordre quelconque, les isomères, on libère les groupes hydroxy protégés et/ou on estérifie un groupe hydroxy libre et/ou on oxyde le groupe 1-hydroxy en acide carboxylique et/ou on hydrogène les doubles liaisons et/ou on saponifie un groupe carboxyle estérifié (R¹ = COOR⁵) et/ou on réduit et/ou on estérifie un groupe carboxyle ( R⁵ = H) et/ou on transforme un groupe carboxyle libre (R⁵ = H) en un amide (R¹ = CONHR⁶) ou on transforme un groupe carboxyle avec une base physiologiquement compatible, en un sel.

5. Préparations pharmaceutiques qui contiennent un ou plusieurs des composés selon la revendication 1.
